# EUROPEAN PATENT APPLICATION

(11) **EP 4 130 263 A1**
(43) Date of publication of application: **08.02.2023**
(21) Application number: 21189707.9
(22) Date of filing: 04.08.2021
(51) Int. Cl.: C12N 15/11, C12N 5/078, C12N 15/113, C12N 15/90

(54) **COMPOUND AND METHOD FOR A SPECIFIC TARGETING OF THE HAX1 GENE**

(71) Applicant: Eberhard Karls Universität Tübingen Medizinische Fakultät, 72074 Tübingen (DE)
(72) Inventor: Skokowa, Julia, 72127 Kusterdingen (DE); Ritter, Malte, 72072 Tübingen (DE); Nasri, Masoud, 72074 Tübingen (DE)
(74) Representative: Witte, Weller & Partner Patentanwälte mbB

(57) **Abstract**

The present invention relates to a 'single guide RNA' (sgRNA) molecule targeting the gene encoding HCLS1 Associated Protein X-1 (*HAX1* gene) in a living being, a 'repair template nucleic acid molecule' for correcting a mutation in the *HAX1* gene in a living being, a vector comprising said sgRNA or said repair template nucleic acid molecule, a composition comprising said sgRNA molecule or repair template nucleic acid molecule, a method *in vitro* for targeting the *HAX1* gene in biological material including genetic material encoding said *HAX1* gene, a method for the prophylaxis and/or treatment and/or examination of a disease in a living being, and a method for editing and/or correcting in a cell a mutant allele of the *HAX1* gene.

## Description

The invention is directed to a 'single guide RNA' (sgRNA) molecule targeting the gene encoding HCLS1 Associated Protein X-1 (*HAX1* gene) in a living being, a 'repair template nucleic acid molecule' for correcting a mutation in the *HAX1* gene in a living being, a vector comprising said sgRNA or said repair template nucleic acid molecule, a composition comprising said sgRNA molecule or repair template nucleic acid molecule, a method *in vitro* for targeting the *HAX1* gene in biological material including genetic material encoding said *HAX1* gene, a method for the prophylaxis and/or treatment and/or examination of a disease in a living being, and a method for editing and/or correcting in a cell a mutant allele of the *HAX1* gene.

### FIELD OF THE INVENTION

The present invention relates to the field of molecular medicine, more particular to the field of genetic engineering applications, preferably to the targeted addressing of disease-associated genes.

### BACKGROUND OF THE INVENTION

Neutropenia is a disorder characterized by an abnormally low concentration of neutrophils in the blood and bone marrow. Neutrophils make up the majority of circulating white blood cells and serve as the primary defense against infections by destroying bacteria, bacterial fragments and immunoglobulin-bound viruses in the blood. People with neutropenia are more susceptible to bacterial infections and, without prompt medical attention, the condition may become life-threatening (neutropenic sepsis). Neutropenia can be acute (temporary) or chronic (long lasting). The term is sometimes used interchangeably with "leukopenia" ("deficit in the number of white blood cells").

Neutropenia can be divided into acquired and congenital. The two main types of the congenital condition are severe congenital neutropenia (CN or SCN) and cyclic neutropenia (CyN).

Cyclic neutropenia is characterized by fluctuating neutrophil counts from normal levels to zero while severe congenital neutropenia is characterized by very low absolute neutrophil count (ANC) (500/ml) observed at birth, maturation arrest of the myelopoiesis in bone marrow at the promyelocyte/myelocyte stage, and early onset of bacterial infections.

Severe congenital neutropenia may be diagnosed by measuring a very low ANC in the blood and examining bone marrow aspirate to identify myeloid maturation arrest. It is usually diagnosed shortly after birth, while diagnosis for CyN is generally raised at different ages, and the main clinical manifestation is recurrent acute stomatologic disorders. Bone marrow examination is often necessary to rule out malignant transformation of hematopoiesis, determine cellularity, assess myeloid maturation, and detect signs of a precise etiology, with cytogenetic bone marrow studies now crucial when CN/SCN is suspected. Antineutrophil antibody assay, immunoglobulin assay (Ig GAM), lymphocyte immunophenotyping, pancreatic markers (serum trypsinogen and fecal elastase) and liposoluble vitamin levels (vitamins A, E and D) are also of interest in assessing CN/SCN and CyN.

A subgroup of patients suffering from severe congenital neutropenia show mutations in the gene encoding HCLS1 Associated Protein X-1 (*HAX1* gene). Patients with autosomal recessive *HAX1* mutations (*HAX1*-CN/SCN) show typical maturation arrest of granulopoiesis in the bone marrow at the stage of promyelocytes/myelocytes. *HAX1* mutations are frequently detected in consanguineous CN/SCN patients, including individuals with Kostmann syndrome.

A majority of *HAX1*-CN/SCN patients harbor the loss-of-function mutation p.W44X, a shift in the reading frame produces a premature stop codon that results in an absence of HAX1 protein expression. A small group of patients has a deletion in exons 4 - 7 of *HAX1,* which affects two isoforms of HAX1 protein expressed in the hematopoietic and neurological tissues causing neurological abnormalities, e.g., developmental delay, cognitive impairment, and/or epilepsy in addition to severe neutropenia.

*HAX1*-CN/SCN patients are at high risk of developing myelodysplastic syndrome (MDS) or acute myeloid leukemia (AML). Co-acquisition of somatic mutations in *CSF3R* and *RUNX1,* as well as trisomy 21, was detected in *HAX1-CN* patients with overt MDS/AML at a similar degree as it was observed in *ELANE-CN* patients.

Currently, neutropenia is treated with the hematopoietic growth factor granulocyte-colony stimulating factor (G-CSF). G-CSF stimulates the production of neutrophils and the delay of their apoptosis. Recombinant G-CSF factor preparations, such as filgrastim, can be effective in people with different forms of neutropenia including severe congenital neutropenia and cyclic neutropenia. The dosage to induce the neutrophil production varies considerably depending on the individual's condition. Overall survival of patients with severe congenital neutropenia is now estimated to exceed 80%, although 10% of CN/SCN patients still die from severe bacterial infections or sepsis. Although G-CSF therapy is successful in preventing mortality from sepsis, chronic G-CSF treatment might deteriorate defective G-CSFR downstream signaling caused by inherited *HAX1* or *ELANE* mutations that ultimately leads to leukemogenic transformation or myelodysplastic syndrome (MDS) in CN/SCN patients.

Hematopoietic stem cell transplant (HSCT) is an alternative, curative therapy for patients who do not respond to G-CSF therapy or who develop acute myeloid leukemia or MDS. However, patients with congenital neutropenia who undergo HSCT are at increased risk of developing infectious complications such as fungal and graft-versus-host disease.

Morishima et al. (2014), Genetic correction of HAX1 in induced pluripotent stem cells from a patient with severe congenital neutropenia improves defective granulopoiesis, Haematologica 99(1), pp. 19-27, the authors describe the correction of a *HAX1* gene deficiency in HAX1-iPS cells by lentiviral transduction with *HAX1* cDNA.

Pittermann et al. (2017), Gene correction of HAX1 reversed Kostmann disease phenotype in patient-specific induced pluripotent stem cells, Blood Adv. 14, pp. 903-914, describe the use of the CRISPR/Cas9 technology to correct in an experimental setup a *HAX1^{W44X}* nonsense mutation in immature myeloid progenitor cells. According to the authors the lentiviral expression of *HAX1* did not repair granulocytic differentiation.

Ritter et al. (2020), Efficient correction of HAX1 mutations in primary HSPCS of severe congenital neutropenia patients using CRISPR/Cas9 gene-editing, European Hematology Association, Abstract EP1479, describe the idea of the application of CRISPR/Cas9 gene editing in combination with rAAV6-based delivery of the template for homology-directed repair (HDR) for the mutated *HAX1* gene in primary bone marrow mononuclear CD34⁺ cells (HSPCs) of *HAX1-CN* patients. However, no particular compound for a use in the proposed approach is disclosed in this document.

Against this background it is an object underlying the invention to provide new compounds and new methods which allow a targeted prophylaxis and/or treatment and/or examination of an *HAX1* gene-associated disorder. Furthermore, a compound is to be provided which allows the addressing of a *HAX1* gene or mutated *HAX1* gene in a specific and targeted manner. In particular such a compound is to be provided which addresses frequently prevalent mutations in the *HAX1* gene.

The present invention satisfied these and other needs.

### SUMMARY OF THE INVENTION

The present invention provides a 'single guide RNA' (sgRNA) molecule targeting the gene encoding HCLS1 Associated Protein X-1 (*HAX1* gene) in a living being for use in the prophylaxis and/or treatment and/or examination of a disease, characterized in that the sgRNA molecule comprises a nucleotide sequence which is selected from the group consisting of SEQ ID NOS: 1-553.

The present invention also provides a 'repair template nucleic acid molecule' for correcting a mutation in the *HAX1* gene in a living being for use in the prophylaxis and/or treatment and/or examination of a disease, characterized in that the repair template nucleic acid molecule comprises the nucleotide sequence of SEQ ID NO: 565.

The object underlying the invention is, in an embodiment, met by the provision of a nucleic acid molecule comprising a nucleotide sequence which is selected from the group consisting of SEQ ID NOS: 1-565, preferably of SEQ ID NOS: 1-554, 556-565.

The inventors have realized that by using the sgRNA molecule according to the invention a specific editing of the *HAX1* gene, preferably a mutated allele thereof, can be achieved. For instance, in an embodiment of the invention the sgRNA molecule comprising or consisting of the nucleotide sequence SEQ ID NO: 552 is configured to specifically target the p.W44X (c.131insA) mutation at exon 2 of the human *HAX1* gene (detailed specification of p.W44X mutation: DNA = NC_000001.11:g. 154273412_154273413insA; RNA = NM_006118.4:c.130_131insA; protein = NP_006109.2:p.(Trp44*)). In this mutation in the HAX1 protein at amino acid position 44 a tryptophan is replaced by any other amino acid. According to the findings of the inventors said mutation can be frequently found in *HAX1* gene-associated disorders such as severe congenital neutropenia (CN/SCN).

The inventors have further realized that by using the repair template nucleic acid molecule according to the invention allows a repair or correction of the mutated and/or edited *HAX1* gene by means of homology-directed repair (HDR). For instance, the repair template comprising sequences homologous to the break sites previously introduced by the sgRNA molecule, serves as a matrix for the cell-owned repair apparatus resulting in the synthesis of the disrupted and/or mutated *HAX1* gene, preferably comprising the p.W44X (c.131insA) mutation at exon 2 of the human *HAX1* gene. In the outcome, the mutated *HAX1* gene is repaired to the non-mutated *HAX1* gene. The repair template nucleic acid molecule includes five silent mutations between the *HAX1* mutation and the cut site to increase gene-editing efficiency by preventing recurrent cutting of correctly edited alleles and premature HDR termination. Thus, the invention allows a causative therapy of a *HAX1* gene-associated disorder. The repair template nucleic acid, in an embodiment of the invention, can be provided and/or delivered as a single stranded oligonucleotide DNA (ssODN). In another embodiment it can also be provided as comprised by a recombinant vector, e.g. an rAAV or rAAV6 vector, or an integrase-deficient lentivirus (IDLV) vector. In yet another embodiment the repair template nucleic acid can be provided and/or delivered encapsuled in nanopartciles.

The inventors were able to demonstrate in an experimental cell system involving primary hematopoietic stem and progenitor cells (HSPCs) expressing mutated HAX1 protein and the use of CRISPR/Cas9 technology that by means of the invention more than 65% correction efficiency with re-expression of HAX1 protein can be achieved. This was in line with markedly improved ex *vivo* granulocytic differentiation and a substantial reduction of H₂O₂-induced apoptosis in *HAX1*-corrected HSPCs.

The findings of the inventors were surprising and could not be expected.

According to the invention 'single guide RNA' (sgRNA) or 'guide RNA' (gRNA) is a component of the CRISPR complex which serves to guide the CRISPR endonuclease Cas9 to its target, i.e. the *HAX1* gene. An sgRNA is a non-coding short ribonucleic acid (RNA) sequence which binds to the complementary target DNA sequence. The sgRNA guides the CRISPR endonuclease enzyme Cas9 to the specific *HAX1* gene location on the DNA, where the CRISPR endonuclease Cas9 mediates a double-strand break.

A 'nucleic acid molecule' as referred to herein includes both deoxyribonucleic acid (DNA) and ribonucleic acid (RNA) molecules, single or double-stranded, comprising concatenated natural and/or chemically modified nucleotides. Modified nucleotide, e.g., include 2'OMe bases which, preferably, increase stability, potency and resistance against nucleases.

According to the invention a *'HAX1* gene' encodes the 'HCLS1-associated protein X-1'. It is a protein that is known to associate with HCLS1, a substrate of Src family tyrosine kinases. It also interacts with the product of PKD2 gene, mutations in which are associated with autosomal-dominant polycystic kidney disease, and with F-actin-binding protein, cortactin. The human variant can be identified as follows: Gene ID (Entrez): 10456; Enseml: ENSG00000143575; uniProt: 000165, Q5VYD6.

According to the invention 'targeting' or 'addressing' refers to a specific and selective interacting with the *HAX1* gene on a molecular level. In an embodiment, targeting includes 'gene editing' or genomic editing being a type of genetic engineering in which DNA is inserted, deleted, modified or replaced in the genome of a living organism. 'Targeting' also includes repairing or correcting a *HAX1* gene, preferably a mutated *HAX1* gene.

In the context of the invention 'gene editing' refers to the modification of the *HAX1* gene, e.g. in a biological cell. Therefore, according to the invention 'gene editing' includes the down regulation, knocking out (KO) or knocking down or the correction of the *HAX1* gene, preferably the mutated *HAX1* gene. In an embodiment of the invention the gene editing results in a targeted knockdown and a specific functional elimination of the expression of the mutated *HAX1* gene, respectively.

According to the invention a 'living being' includes any beings incorporating a *HAX1* gene, including mammals, preferably human beings.

The problem underlying the invention was herewith fully achieved.

In an embodiment the sgRNA molecule and/or repair template nucleic acid molecule of the invention is configured for a use in the prophylaxis and/or treatment and/or examination of a disease, preferably an *HAX1*-associated disease, further preferably of congenital neutropenia, and further preferably of severe congenital neutropenia (CN/SCN), and/or cyclic neutropenia (CyN).

This measure has the advantage that the invention can be used for addressing a *HAX1*-associated disease, in particular CN/SCN or CyN, but also emphysema or emphysematous changes, in a causative manner.

In another embodiment said disease is myelodysplastic syndrome (MDS) and/or myeloid leukemia (AML).

*HAX1-CN* patients are at high risk of developing myelodysplastic syndrome (MDS) or acute myeloid leukemia (AML). With this embodiment, the invention also provides an effective remedy for the two aforementioned diseases.

Another subject-matter of the invention is a vector comprising the sgRNA molecule of the invention and/or comprising the repair template nucleic acid molecule of the invention.

According to the invention, a 'vector' includes any DNA molecule used as a vehicle to artificially carry a nucleic acid molecule, such as an sgRNA, into another cell, where it can be replicated and/or expressed. Viral vectors are of particular preference as they are characterized by embodying the viruses' specialized molecular mechanisms to efficiently transport genomes inside the cells they infect. The vector may or may not be, in a preferred embodiment of the invention, an adeno-associated vector (AAV).

The features, characteristics, advantages and embodiments described for the sgRNA molecule of the invention and/or the repair template nucleic acid molecule according to the invention also apply to the vector according to the invention.

In an embodiment of the invention said vector is a recombinant adeno-associated vector (rAAV), preferably with AAV6 serotype (rAAV6).

This measure has the advantage that such a vector is used which, when using the CRISPR/Cas9 technology, results in particularly high spreading of the CRISPR/Cas9 components, hence superior gene editing.

The invention also relates to a composition comprising the sgRNA molecule according to the invention and/or the repair template nucleic acid molecule according to the invention and/or the vector according to the invention.

The features, characteristics, advantages and embodiments described for the sgRNA molecule of the invention and/or the repair template nucleic acid molecule according to the invention also apply to the vector according to the invention.

In an embodiment of the invention said composition further comprises a CRISPR associated protein 9 (Cas9) and/or a vector encoding Cas9, preferably *Streptococcus pyogenes* Cas9 (S.p. Cas9), further preferably S.p. Cas9 V3, and highly preferably S.p. HiFi Cas9 V3.

This measure establishes the requirements for applying the CRISPR/Cas9 technology. Cas9 (CRISPR associated protein 9, formerly called Cas5, Csn1, or Csx12), a 160 kilodalton protein, refers to an enzyme which cleaves the phosphodiester bond within a polynucleotide chain, which is a component of a CRISPR (Clustered Regularly Interspaced Short Palindromic Repeat) complex. The Cas9 is, therefore, capable to specifically bind to the sgRNA which directs the endonuclease to the target nucleic acid. *Streptococcus pyogenes* CRISPR Cas9 (S.p. Cas9) is a well-characterized Cas9 orthologue, which has proven itself in the field of gene editing. S.p. Cas9 V3 is a recombinant endonuclease purified from an *E. coli* strain containing a nuclear localization sequence (NLS) and a C-terminal 6-His tag. S.p. HiFi Cas9 is a Cas9 variant with improved specificity based on reduced off-target effects, while preserving high on-target activity. The latter is, therefore, ideal for approaches that are sensitive to off-target events and require a high level of editing efficiency. S.p. Cas9 V3 and S.p. HiFi Cas9 are obtainable at Integrated DNA Technologies, Inc., Coralville, Iowa, USA. Other small Cas9 have been recently discovered, such as originating from *Staphylococcus aureus, Staphylococcus auricularis, Campylobacter jejuni* or *Neisseria meningitidis,* and are likewise suitable.

In another embodiment of the invention said composition is a pharmaceutical composition comprising a pharmaceutically acceptable carrier.

A "pharmaceutical composition" is a composition suitable for an administration to an animal and/or human being in a medical setting. Preferably, a pharmaceutical composition is sterile and produced according to GMP guidelines.

'Pharmaceutically acceptable carriers' or excipients are well known in the art and include, for example, nanocarriers, nanovectors, aqueous solutions such as water or physiologically buffered saline or other solvents or vehicles such as glycols, glycerol, oils such as olive oil or injectable organic esters. In preferred embodiments, when such pharmaceutical compositions are for human administration, e.g., for parenteral administration, the aqueous solution is pyrogen-free, or substantially pyrogen-free. The excipients can be chosen, for example, to effect delayed release of an agent or to selectively target one or more cells, tissues or organs. The pharmaceutical composition can be in dosage unit form such as an injection, tablet, capsule (including sprinkle capsule and gelatin capsule), granule, powder, syrup, suppository, or the like. The composition can also be present in a solution suitable for topical administration. Alternatively, the pharmaceutical composition can be present in form of an aerosol administrable by inhalation.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Suitable pharmaceutical carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

The pharmaceutical composition as well as the methods to be explained below of the present invention may be utilized to treat a living being in need thereof. In certain embodiments, the living being is a mammal such as a human, or another non-human mammal.

In an embodiment to the composition according to the invention the latter is configured for the prophylaxis and/or treatment and/or examination of a disease, preferably of an *HAX1* gene-associated disease, further preferably congenital neutropenia, further preferably severe congenital neutropenia (CN/SCN), and/or cyclic neutropenia (CyN), and/or myelodysplastic syndrome (MDS) and/or myeloid leukemia (AML).

The features, characteristics, advantages and embodiments described for the sgRNA molecule of the invention and/or the repair template nucleic acid molecule according to the invention and the vector according to the invention also apply to the composition according to the invention.

Another subject-matter of the invention is a method *in vitro* for targeting the *HAX1* gene in biological material including genetic material encoding said *HAX1* gene, comprising the step of introducing the sgRNA molecule according to the invention and/or the repair template nucleic acid molecule according to the invention and/or the vector according to the invention, and or said composition according to the invention into said biological material, preferably said editing is made *via* CRISPR/Cas9 technology.

According to the invention, 'biological material including genetic material encoding said *HAX1* gene' includes biological cells, tissues and parts of organisms.

In an embodiment of the invention said biological material comprises hematopoietic stem and progenitor cells (HSPCs).

This measure has the advantage that the *HAX1* gene is targeted in such biological material where the mutation is pathologically manifested. E.g. in CN/SCN patients HSPCs show a diminished granulocytic differentiation which, according to the findings of the inventors, can be restored by the nucleic acid molecule according to the invention.

Another subject-matter of the invention relates to a method for the prophylaxis and/or treatment and/or examination of a disease in a living being, preferably selected from the group consisting of congenital neutropenia, preferably severe congenital neutropenia (CN/SCN), cyclic neutropenia (CyN), myelodysplastic syndrome (MDS), myeloid leukemia (AML),comprising a step of targeting or editing the *HAX1* gene in said living being by introducing the sgRNA molecule of the invention and/or the repair template nucleic acid molecule of the invention and/or the vector of the invention, and or said composition of the invention into said biological material, preferably said targeting or gene editing is made *via* CRISPR/Cas9 technology.

The present invention also provides for a method for editing and/or correcting in a cell a mutant allele of the *HAX1* gene, preferably having a mutation associated with congenital neutropenia, severe congenital neutropenia (CN/SCN) or cyclic neutropenia (CyN), and which *HAX1* gene of said cell is mutated at nucleotide position c.131insA, and/or which *HAX1* gene product is mutated at amino acid position p.W44X, the method comprising:
- introducing to the cell a composition comprising:
   - a CRISPR associated protein 9 (Cas9) or a sequence encoding Cas9;
   - an sgRNA molecule comprising a nucleotide sequence which is selected from the group consisting of SEQ ID NOS: 1-553;
wherein a complex of the Cas9 and the sgRNA molecule affects a double strand break in the mutant allele of the *HAX1* gene.

In an embodiment of said method said composition further comprises a repair template nucleic acid molecule comprising the nucleotide sequence of SEQ ID NO: 565, wherein said repair template nucleic acid molecule affects the repair of the mutant allele of the *HAX1* gene.

The present invention provides for a modified cell obtained by the aforedescribed method of the present invention.

The present invention provides for a method of preparing *in vitro* or ex *vivo* a composition comprising modified cells, the method comprising:
a) isolating or providing HSPCs from cells obtained from a living being, preferably a human being, with a *HAX1* gene mutation related to CN/SCN or CyN or MDS or AML, and, preferably, which *HAX1* gene is mutated at nucleotide position c.131insA, and/or which *HAX1* gene product is mutated at amino acid position p.W44X,
b) introducing to the cells of step (a) a composition comprising:
   a CRISPR nuclease or a sequence encoding the CRISPR nuclease; and an sgRNA molecule comprising a nucleotide sequence selected from the group consisting of SEQ ID NOS:1-553,
   wherein a complex of the CRISPR nuclease and the sgRNA molecule affects a double strand break in the mutant *HAX1* gene in one or more cells, so as to inactivate the mutant *HAX1* gene in one or more cells thereby obtaining modified cells; optionally
c) culture expanding the modified cells of step (b), wherein the modified cells are capable of engraftment and giving rise to progeny cells after engraftment.

In an embodiment of the aforementioned method according to the invention said composition further comprises a repair template nucleic acid molecule, preferably comprising the nucleotide sequence of SEQ ID NO: 565, wherein said repair template nucleic acid molecule affects the repair of the mutant allele of the *HAX1* gene.

In another embodiment of said method according to the invention the latter comprises the additional following step d) where the cells of step (b) or step (c) are administered to a living being for treating the CN/SCN or CyN or MDS or AML in the subject.

Therefore, the present invention also provides for a method of treating a living being, preferably a human, afflicted with CN/SCN or CyN, comprising administration of a therapeutically effective amount of the modified cells.

The features, characteristics, advantages and embodiments described for the nucleic acid molecule, vector, and composition according to the invention also apply to the afore-referenced methods according to the invention.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

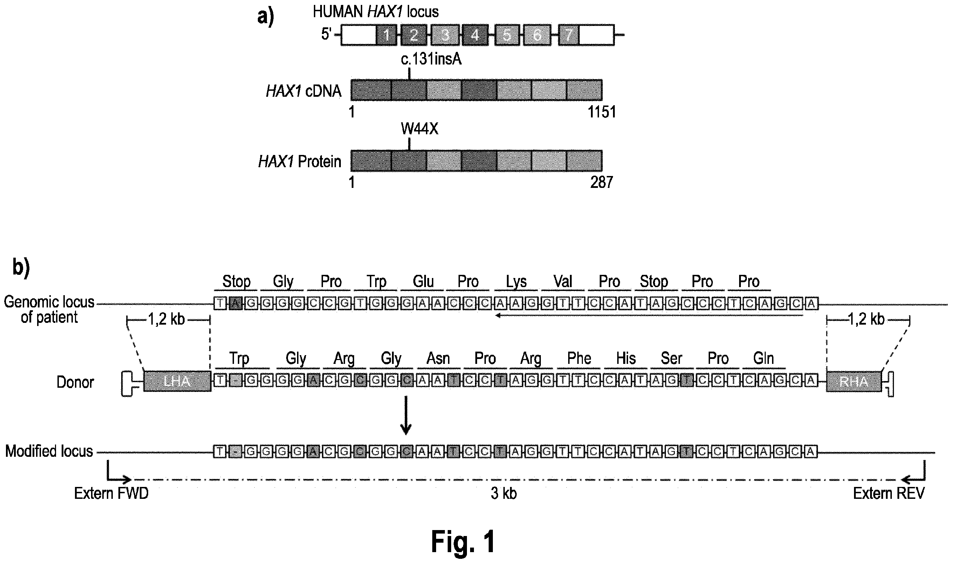

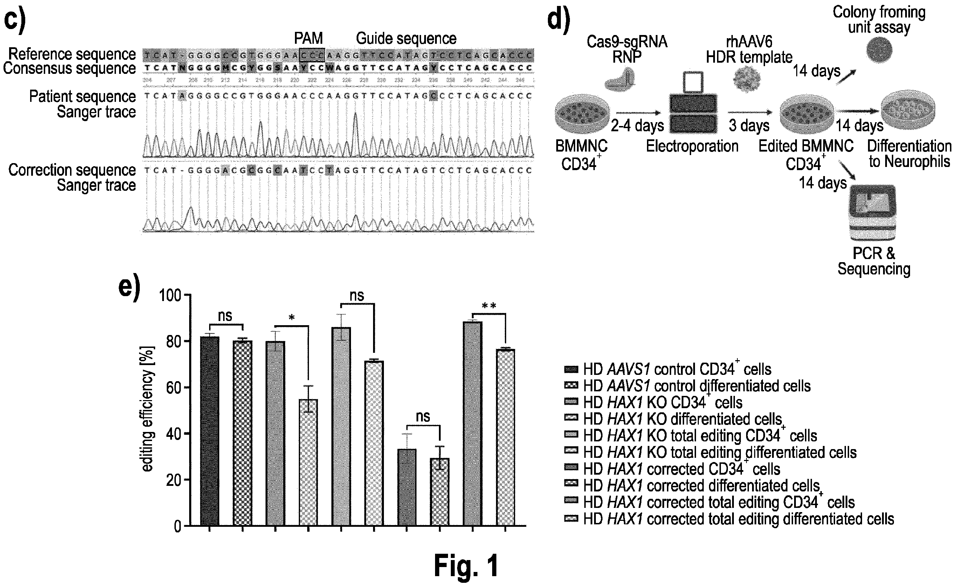

- Fig. 1: Experiment design and editing efficiencies in healthy donor HSPCs. A, Schematic depiction of *HAX1* gene, cDNA, and protein with the indication of CN causing mutation p.W44X. B, Depiction of the design of adeno associated virus 6 DNA donor template and sgRNA targeting the *HAX1* gene. The mutation causing adenosine is marked in red in the patient sequence sanger trace and the SNP is marked in blue. Silent mutations that are introduced during correction are marked in their respective colors. C, Sequencing traces of exemplary patient trace and the correction template aligned to reference sequence. Differences from the reference sequence are marked in pink in the consensus sequence. D, Schematical depiction of experimental workflow, starting with HSPC culture, delivery of CRISPR/Cas9 RNP by electroporation, transduction with AAV HDR template, genetic analysis, and evaluation of granulocytic differentiation. E, Gene editing efficiency in healthy HSPCs obtained 72 hours post-editing and 14 days after differentiation (n = 2). * = p < 0.05, ** = p < 0.01

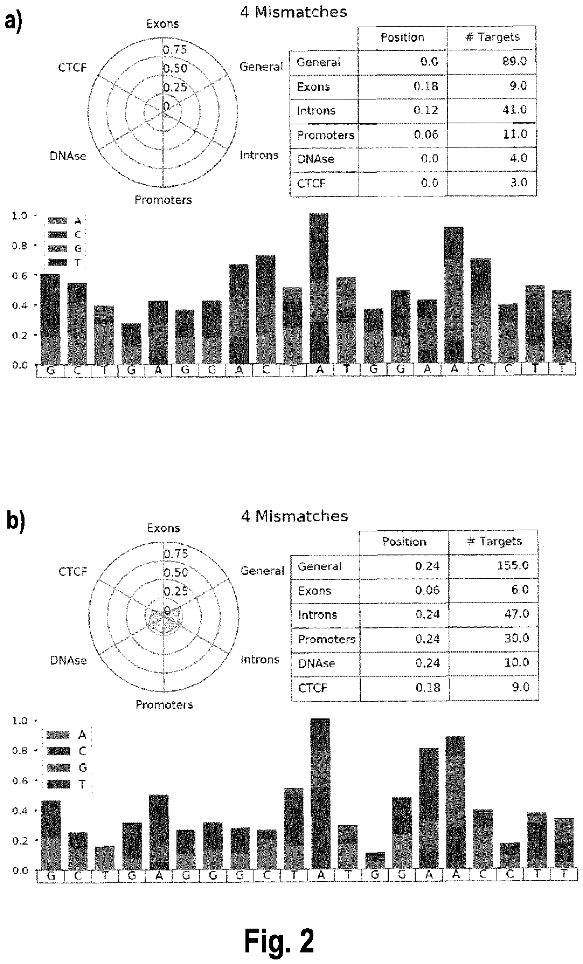

- Fig. 2: In silico off-target prediction. A, Off-target prediction of sgRNA HD shows the absolute number of off-targets for up to four mismatches based on genome hg 19. Furthermore, the relative frequency of off-targets is compared to all other potential guide RNAs in a distance of 30 base pairs upstream and downstream of the targeted mutation. The stacked diagram shows the frequency at which off mismatches are observed at each position in the guide RNA. B, Off-target comparison of the sgRNA containing the matching base as the SNP rs. The analysis was conducted on genome hg 19. The guide is compared to the same list of gRNAs that guide the Cas9 to induce a double-strand break within 30 base pairs of the disease-causing mutation.

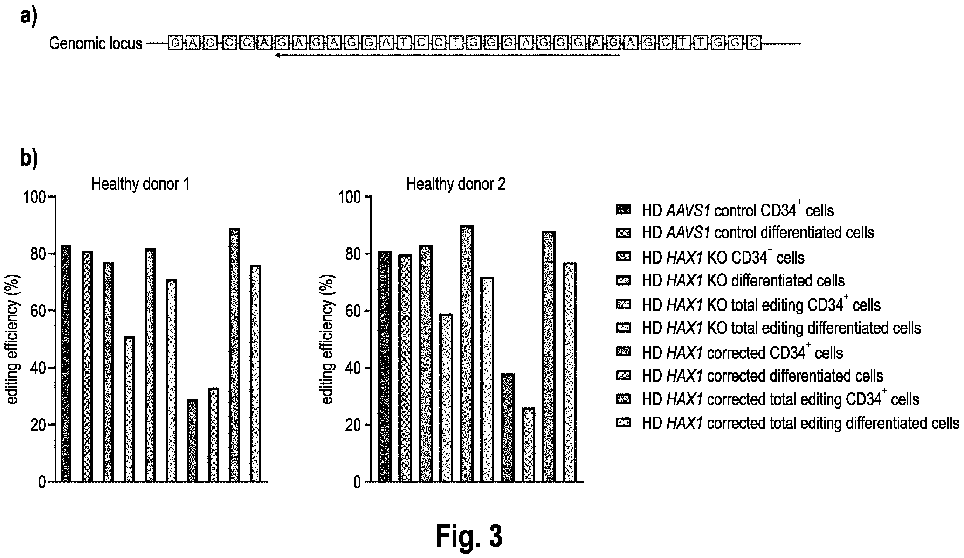

- Fig. 3: AAVS1 sgRNA sequencing and gene-editing efficiency. A, Sequence of guide RNA used for AAVS1 locus. B, The gene-editing efficiencies in each condition and for all time points shown individually for each healthy donor. Efficiencies were determined by deconvolution of Sanger sequencing traces with the ICE algorithm.

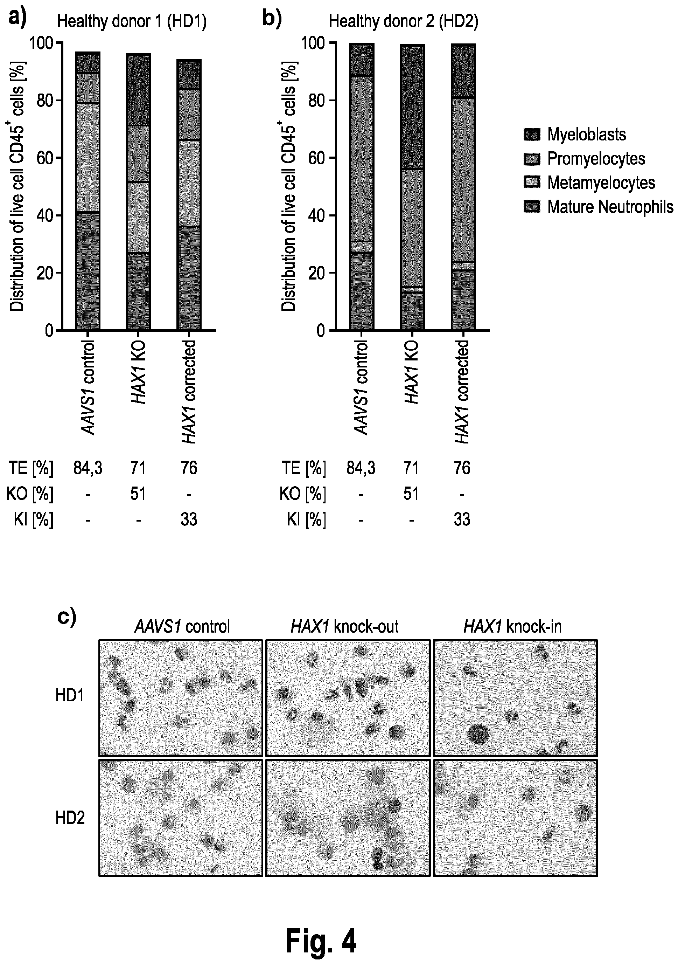

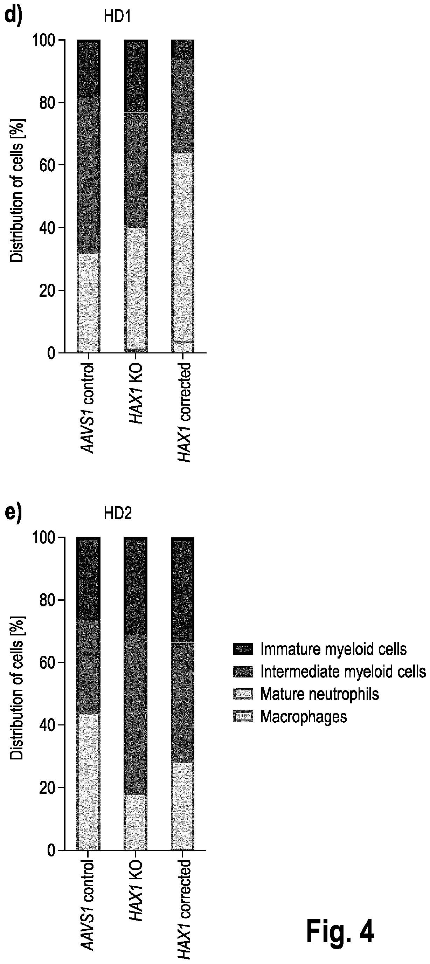

- Fig. 4: Differentiation of *HAX1* knockout mimics *HAX1-CN.* A & B, Flow cytometry analysis of granulocytic differentiation on day 14 of ex *vivo* differentiation presented as distribution of live cells: myeloblasts (CD45⁺CD34^{DIM/-}CD33⁺CD66b⁻), promyelocytes (CD45⁺CD34^{DIM/-}CD33^{+/high}CD66b⁺CD11b^{-/+}), metamyelocytes (CD45⁺CD34⁻CD33^{DIM/-}CD66b⁺CD11b⁺CD16⁻) and neutrophils (CD45⁺CD34⁻CD33^{DIM/-}CD66b⁺CD11b⁺CD16⁺) according to expression of differentiation markers. Each healthy donor is depicted separately, including gene-editing efficiencies in *in vitro* differentiated myeloid cells at day 14 of differentiation (n = 2). C, Exemplary images of cytospins prepared from cells on day 14 of ex *vivo* neutrophil differentiation at 63x magnification. D & E, Distribution of immature myeloid cells, intermediate myeloid cells, mature neutrophils, macrophages, and dead cells shown as percentages.

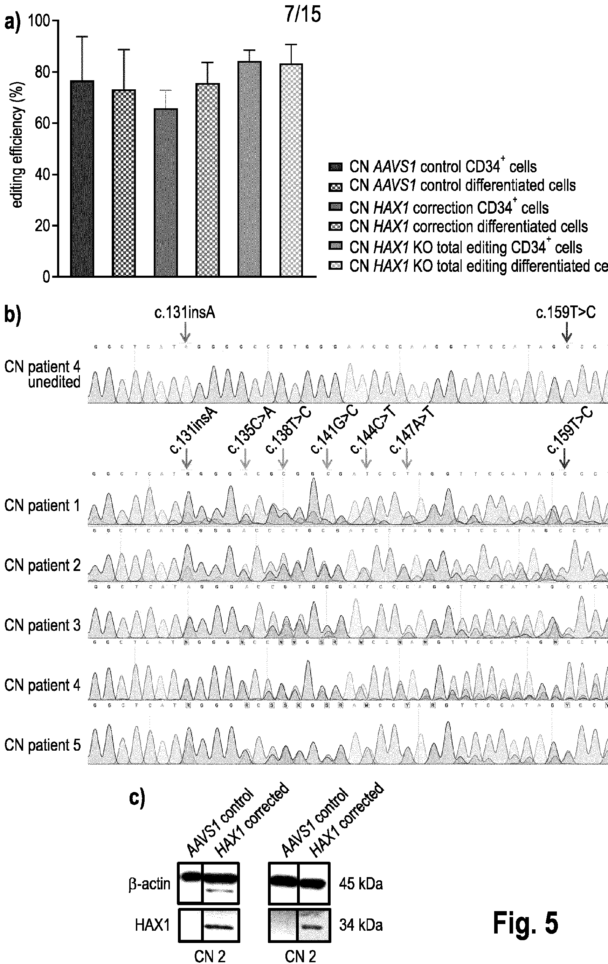

- Fig. 5: Gene-editing efficiency in *HAX1-CN* patients HSPCs. A, Gene-editing efficiency in *HAX1-CN* HSPCs, 72 hours post electroporation and after 14 days ex *vivo* differentiation (n = 5) measured by deconvolution from sanger sequences by ICE algorithm. B, Sanger sequencing traces of all 5 patient HSPCs corrected in this work. The red arrow indicates the adenosine that causes the stop codon in *HAX1* responsible for losing HAX1 protein. Green arrows mark silent mutations introduced by the HDR template. The blue arrow marks SNP rs13796 (dbSNP build 150) that all patients with p.W44X mutation carry. C, Protein expression analysis through western blot technique showed expression of HCLS1-associated protein X-1 after editing. Cells analyzed were differentiated to neutrophils prior to analysis. The analysis was performed for two independent patients.

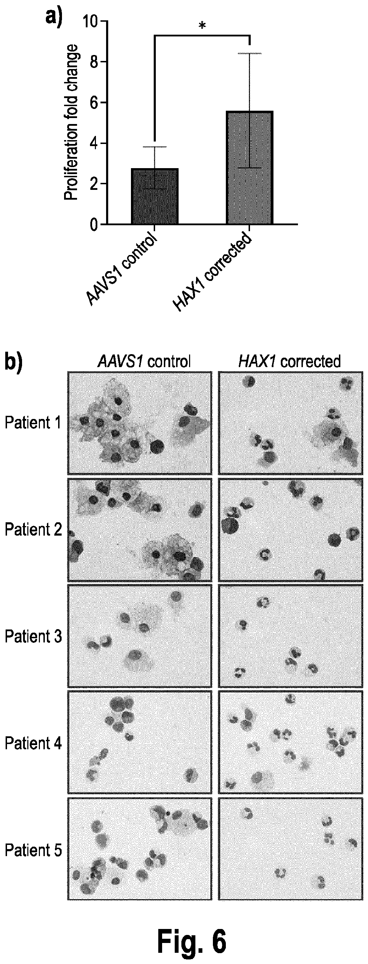

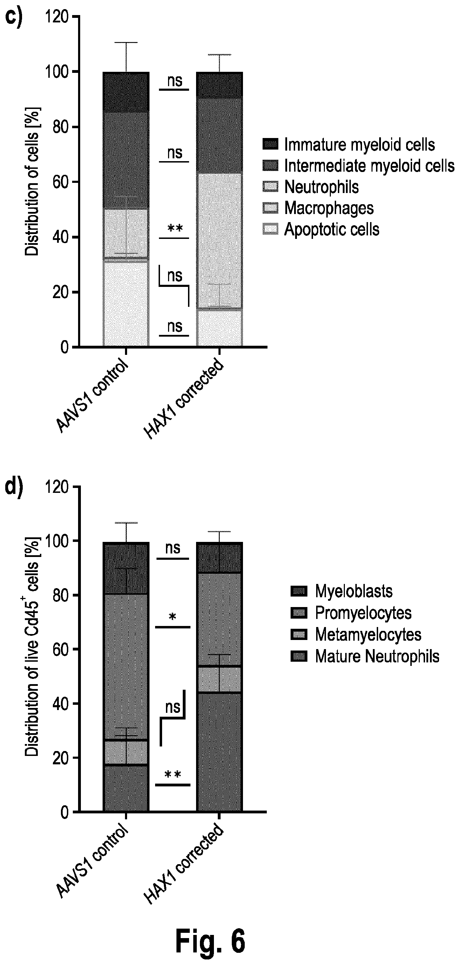

- Fig. 6: Evaluation of ex *vivo* differentiation of corrected cells. A, Proliferation of *AAVS1* control edited and *HAX1* corrected cells from day 1 until day 14 of liquid culture expressed as fold change of cells counted at the respective days. Statistical significance was determined by student's t-test (n = 5). B, Representative images of Wright-Giemsa stained cytospins of differentiated HSPCs on day 14 of differentiation. Cells from each patient and each condition are shown (n = 5). Differential composition of cells 14 days post *in vitro* differentiation displayed as percentage of all counted cells from Wright-Giemsa stained cytospins. C, Flow cytometry analysis of granulocyte differentiation markers after 14 days of ex *vivo* differentiation are presented as distribution of live cells: myeloblasts (CD45⁺CD34^{DIM/-}CD33⁺CD66b⁻), promyelocytes (CD45⁺CD34^{DIM/-}CD33^{+/high}CD66b⁺CD11b^{-/+}), metamyelocytes (CD45⁺CD34⁻CD33^{DIM/-}CD66b⁺CD11b⁺CD16⁻) and neutrophils (CD45⁺CD34⁻CD33^{DIM/-}CD66b⁺CD11b⁺CD16⁺) according to expression of differentiation markers. * = p < 0,05, ** = p < 0,01.

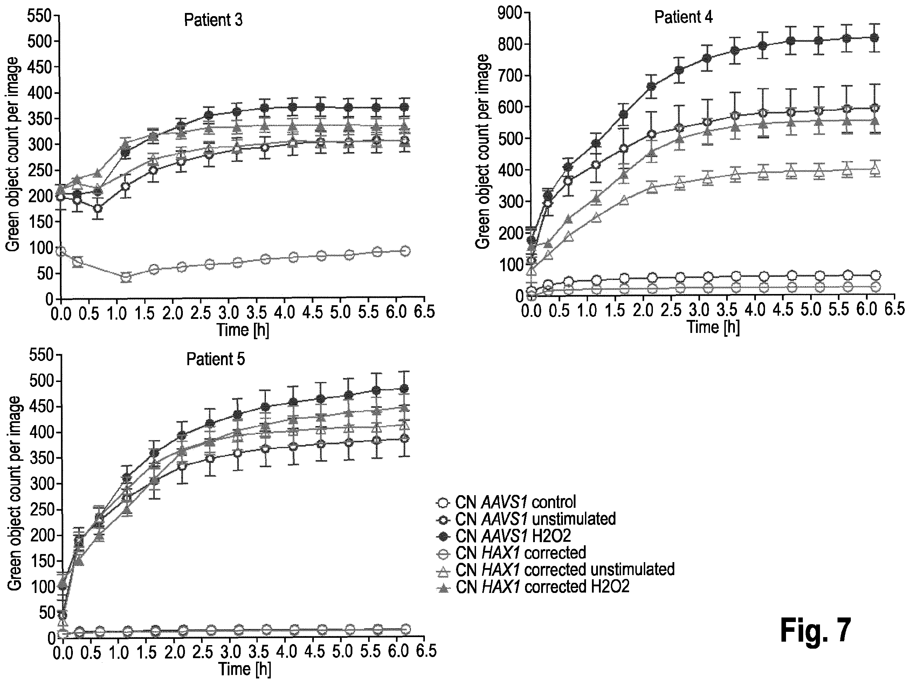

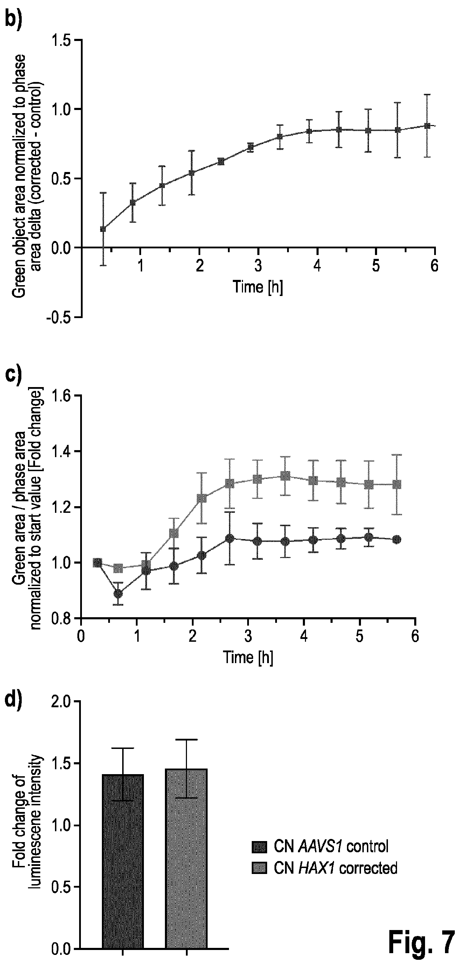

- Fig. 7: The functionality of ex *vivo* generated neutrophils from HAX1-CN patients. A, Apoptosis induction upon oxidative stress in control edited and corrected HAX1 patient cells (n = 3). Apoptosis is detected by Caspase3/7 cleavage that induces a green fluorescence and is measured by live-cell imaging. Data for each patient is shown separately with mean error of 3 technical replicates per patient. B, Phagocytosis of pHrodo green E. coli bioparticles in differentiated cells was measured by live-cell imaging. Phagocytosis is displayed as the arithmetic difference between corrected and control differentiated cells number of green fluorescent cells. Error bars depict standard error for replicates. C, Formation of neutrophil extracellular traps (NETs) was determined by DNA staining in live-cell imaging. The green area was normalized to the phase area at the experiment start, and the results are depicted as fold change from unstimulated to PMA stimulated cells. D, ROS production capability of *in vitro* derived myeloid cells as fold change from unstimulated to f-MLP stimulated myeloid cells. ROS levels are measured by luminescence with the ROS-Glow Kit (Promega) (n = 2).

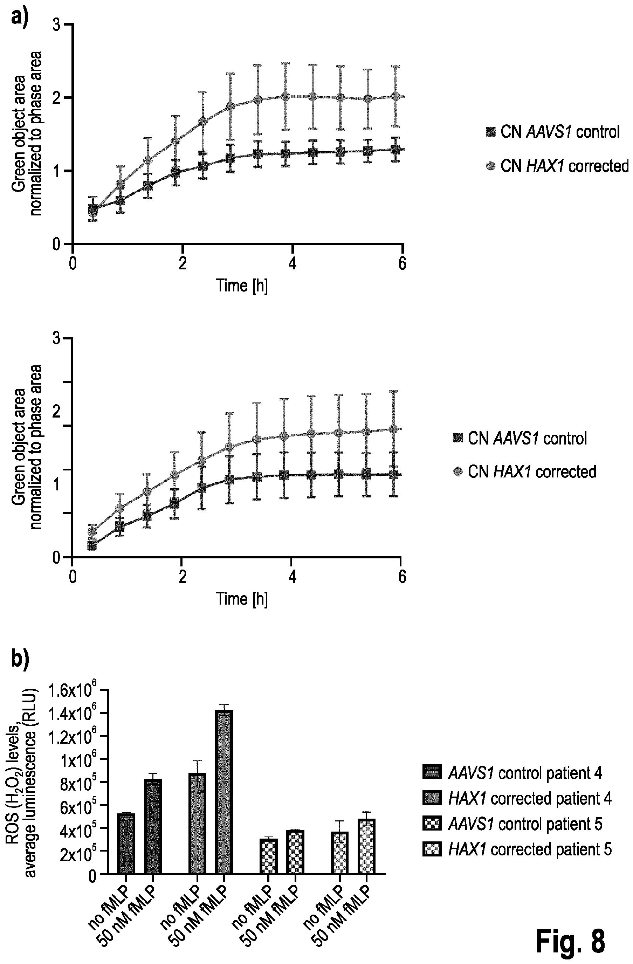

- Fig. 8: Live cell analysis of phagocytosis of *E. coli* bioparticles. A, Phagocytosis of pH-rodo green *E. coli* bioparticles is shown for ex *vivo* generated neutrophils for two patients. Shown are the average and standard error of technical triplicates of control edited cells and corrected cells as green object area normalized to phase area for each patient.

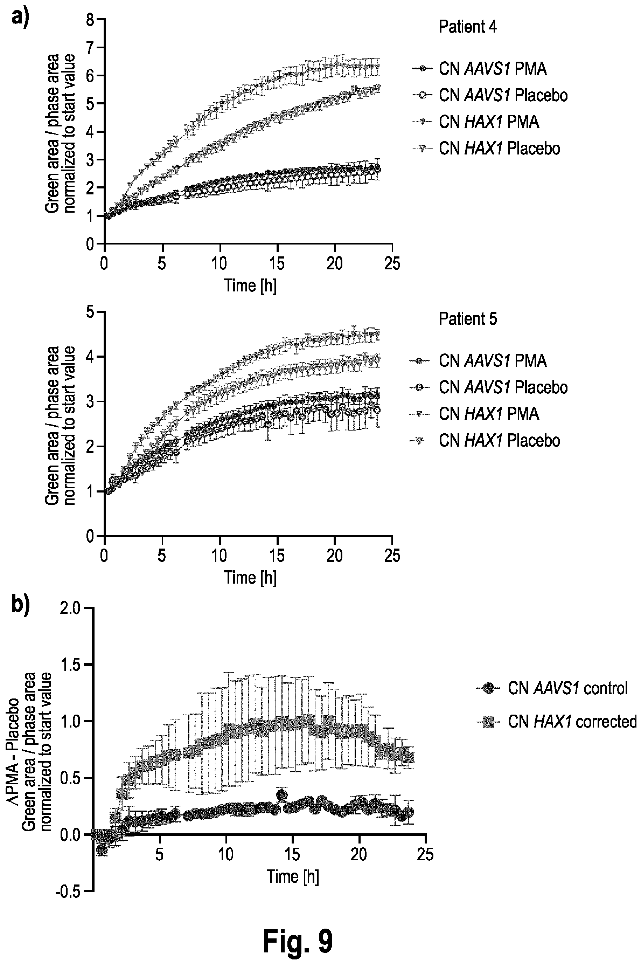

- Fig. 9: Live cell analysis of neutrophil extracellular trap formation. A, NET formation by differentiated cells upon stimulation with PMA is shown per patient as total Phase Object Area (µm²/Well) measured at the bottom. The measurements are shown for 24 hours, at which time the equilibrium is reached. The Experiment was carried out for two donors, and each condition per donor was performed in triplicates. B, The difference between PMA stimulated and placebo-treated cells is shown. Error bars represent the standard error per point. (n = 2).

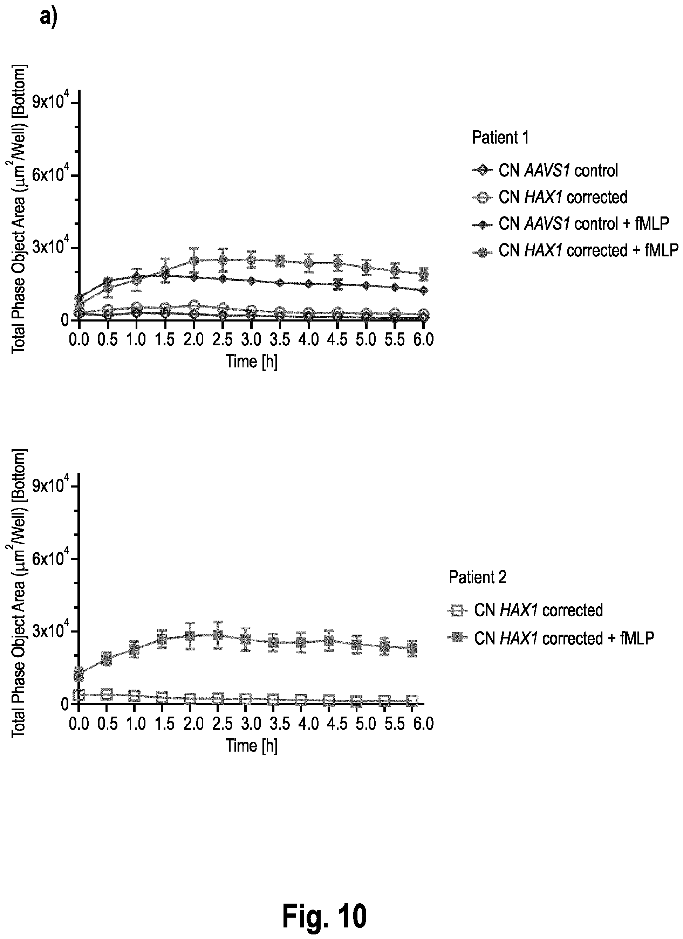

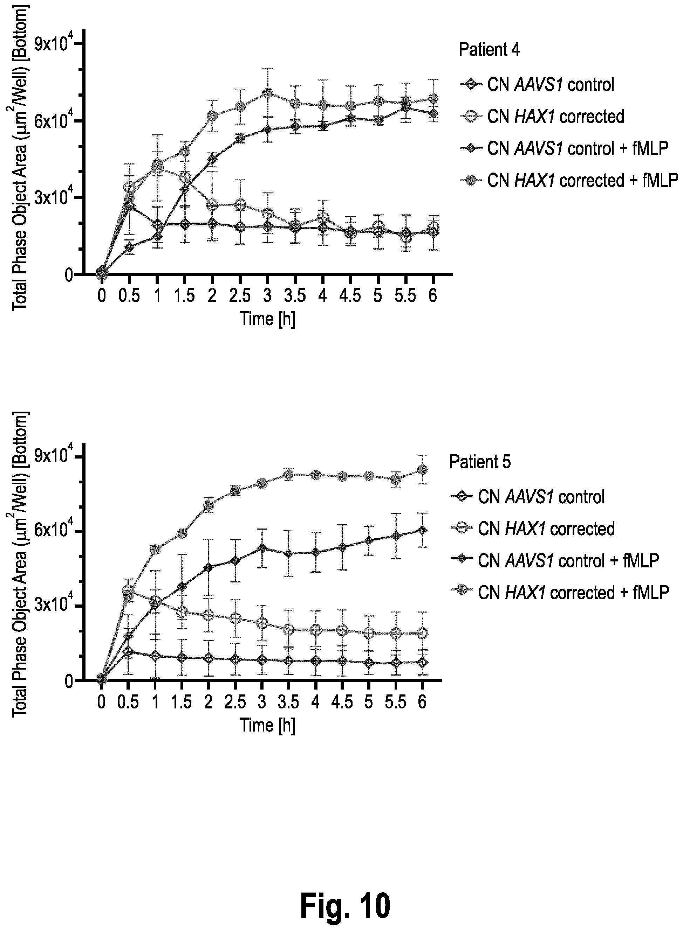

- Fig. 10: Live cell analysis of chemotaxis of differentiated cells. A, Chemotaxis of differentiated cells is shown per patient as total Phase Object Area (µm²/Well) measured at the bottom. The measurements are shown for 6 hours, at which time the equilibrium is reached. As a chemoattractant, the inventors used fMLP at 50 nM. The Experiment was carried out for four donors, and each condition per donor was performed in triplicates.

### EXAMPLES

### 1. Introductory note

Recent improvements in the CRISPR/Cas9 gene-editing platform have unlocked an unprecedented efficiency to precisely edit the genome of human cells, including HSPCs, while being accessible to a broad range of translational laboratories. Besides being an excellent tool to knockout disease-causing genes, restore the reading frames, or knock-in whole genes, it can also be used to specifically change, insert or delete single nucleotides or a set of nucleotides that cause disease. The intentional editing of bases in HSPCs is achieved by triggering homology-directed repair (HDR) through the introduction of a double-strand break with Cas9 and the subsequent repair with an HDR template homologous to the break sites. To this end, the combination of CRISPR/Cas9 with a repair template delivered by recombinant adeno associated virus serotype 6 (rAAV6) has proven to be highly efficient and reproducible for ex *vivo* gene therapy using the autologous settings of inherited bone marrow failure syndromes. The FDA has recently approved the first clinical trial utilizing this approach for sickle cell disease. In the present application, the inventors show that CRISPR/Cas9-mediated introduction of indels into exon 2 of the *HAX1* gene of HSPCs derived from healthy donors led to blocked granulocytic differentiation ex *vivo,* resembling a CN-phenotype. This phenotype could be corrected with a *HAX1* HDR repair template delivered by rAAV6. Based on this model, the inventors established an ex *vivo* gene-therapy approach to correct the mutation p.W44X (c.131insA) in the *HAX1* gene, restoring granulocytic differentiation in cells derived from CN patients. Being that the *HAX1* mutation p.W44X is highly reported in *HAX1-CN* patients, the inventors' approach may provide a broad application in this group of patients.

### 2. Material and methods

### Cloning of HAX1 genomic sequence

A 3009 bp fragment of genomic DNA was amplified using HS Phusion II (Thermo Fisher Scientific #F549L) and *HAX1* AAV template primers (Supplementary Table 1). Using a nested PCR approach and phosphorylated primers phos-*HAX1*-*AAV* HDR (Table 1), a 2406 bp fragment was produced from the 1^{st} PCR product. This PCR product was cloned between the ITR ends of the pAAV-CMV plasmid (Takara #6234) after the CMV expression cassette was removed by enzymatic digestion with EcoRV (NEB #R0195S) and BgIII (Thermo Fisher Scientific # D0083), and the ends were blunted with T4 DNA polymerase (Thermo Fisher Scientific # EP0061). Blunt-end cloning was carried out with T4 DNA Ligase (Thermo Fisher Scientific #EL0014). After ligation, the plasmids were treated with AT-dependent DNase (Lucigene E3101K). Subsequently, the newly created plasmids were transformed into Stellar cells (Takara #636763). For selected clones, the insert was fully sequenced using Sanger sequencing primers. Using site-directed mutagenesis (Agilent #200521), the inventors introduced five silent mutations that preserve the protein sequence in translation but inhibit double-strand breaks induced by Cas9 in the corrected allele (Figure 1B, C). The generated fully sequenced plasmid (AAV-HAX1-HDR-p.R50) was further used for AAV particle production.

### Recombinant adeno-associated virus production

The adeno-associated virus was produced as described by Bak et al. (2018), CRISPR/Cas9 genome editing in human hematopoietic stem cells. Nature Protocols 13(2), pp. 358-376, or ordered from Vigene Biosciences. Briefly, HEK293T cells (density between 70-90 %) were transduced with plasmids AAV-HAX1-HDR-p.R50 and PDGM6 (Addgene #110660) using polyethyleneimine (PEI). After 48 hours, transduced cells were harvested by adding 6.25 mM EDTA to the medium. Harvested cells were lysed by repeated thawing at 37°C and freezing in a dry ice ethanol bath with subsequent benzonase (Biovision #7680) digestion. Lyzed cells were centrifuged to eliminate debris. Viral particles were purified from the supernatant by iodixanol gradient ultracentrifugation. The band containing viral particles was dialyzed overnight and titrated by real-time PCR (Takara #6233).

### sgRNA and HAX1 correction template design

The inventors selected specific single-guide RNAs (sgRNAs) for the *HAX1* gene (including cut site: chr1 [GCTGAGGACTATGGAACCTT (HAX1 Ex 2; SEQ ID NO: 553), +154273431: -154273432; Table 1], NM_006118.4 Exon 2, 244 bp; NP_006109.2 p.R50). All *HAX1-CN* patients' cells used in this study presented an SNP rs13796 (dbSNP build 150) in the binding site of our sgRNA. The inventors used, in their exemplary experiments, a patient-specific sgRNA with the cut site (GCTGAGGGCTATGGAACCTT (sgHAX1.PS-SNP; SEQ ID NO: 552; Table 1) containing this SNP. An sgRNA targeting AAVS1 safe harbor was selected from a previous study (cut site: chr19 [CTCCCTCCCAGGATCCTCTC (AAVS1; SEQ ID NO: 554, +55115580: - 55115581; Table 1]). Chemically modified sgRNAs were obtained from IDT.

### CRISPR/Cas9 RNP delivery and AAV transduction

After 2-4 days of HSPCs expansion, the sgRNA (sgHAX1.PS-SNP; SEQ ID NO: 552) and sp.Cas9 V3 (IDT #1081059) were delivered by electroporation with Amaxa 4D Nucleofector (Lonza #AAF-1002X) and the P3 primary kit (#V4XP-3024) using the program CA-137. Within one hour after electroporation, the cells were transduced at an MOI of 100.000 with the repair template vector (SEQ ID NO: 565) or control AAV and cultured for 48 additional hours.

### Gene-editing analysis

Gene-editing efficiency was measured 72 hours post-electroporation and transduction and on day 14 after differentiation. Genomic DNA was extracted using a quick-extract solution (Lucigen #QE09050). Using HS Phusion II (Thermofisher Scientific #F549L) and *HAX1* AAV template primers (Table 2), a 3007 bp fragment of genomic DNA was amplified and sequenced using Sanger sequencing. Control editing in the *AAVS1* locus was measured by amplifying a 655 bp fragment (AAVS1 primer 1; Table 2). Editing efficiency was calculated from the Sanger traces using ICE synthego. *HAX1* locus was amplified with primers outside of the repair template to detect genomic sequences only.

### HSPC isolation and culture

CD34⁺ hematopoietic stem and progenitor (HSPCs) were isolated from bone marrow biopsies. Briefly, mononuclear cells were obtained through Ficoll gradient (GE Healthcare #17-1440-03) centrifugation and subsequently used for CD34⁺ purification using magnetic beads (Miltenyi #130-046-703). CD34⁺ cells were cultured in STEM SPAN SFEM II supplemented with 20 ng/ml interleukin-3 (IL-3), 50 ng/ml stem cell factor (SCF), 20 ng/ml interleukin-6 (IL-6), 20 ng/ml thrombopoietin (TPO), 50 ng/ml FSM-like tyrosine kinase 3 ligand (Flt-3L), 2mM L-Glutamin, 100 U/ml Penicillin and 0.1 mg/ml Streptavidin at a density from 2.5 x 10⁵ to 5 x 10⁵ cells/ ml.

### Ex vivo neutrophilic differentiation analysis

Gene-edited cells were differentiated into neutrophils by culturing them in RPMI 1640 Glutamax supplemented with 10% FCS, 2 mM L-Glutamate, 5 ng/ml SCF, 5 ng/ml IL-3, 5 ng/ml GM-CSF, 1 ng/ml G-CSF, 100 U/ml penicillin and 0.1 mg/ml streptavidin at a density of 5 x 10⁵/ml. Half of the Medium was exchanged every other day. After 7 days, cells were counted, centrifuged, and seeded in RPMI 1640 Glutamax supplemented with 10% FCS, 2 mM L-Glutamate, 1 ng/ml G-CSF, 100 U/ml penicillin, and 0.1 mg/ml streptavidin. Cell were seeded again at the same density and frequency of media exchange was kept the same until day 14.

### Cell morphology analysis

Cytospins were prepared by centrifuging 1 x 10⁴ cells in 100µl PBS for 3 minutes at 250 RPM onto glass microscope slides. After air-drying, slides were stained with May-Grünwald and Giemsa solution. Cell morphology was estimated at 63x magnification, and 100 cells were counted per slide using a Nikon Eclipse TS100.

### Myeloid/granulocytic cell surface markers analysis

Differentiated cells were stained with antibodies against CD45 (Biolegend BV510 #304036), CD11b (Biolegend APC-Cy7 #301322), CD66b (Biolegend FITC #305104), CD16 (BD Bioscience APC #561248), CD33 (Biolegend BV421 #303416), CD34 (BD Bioscience PECy-7 #128618) and live/dead cell exclusion reagent (BD Bioscience 7AAD #559925). Cells were incubated in a final volume of 100 µl of PBS supplemented with 0,5% BSA (FACS-Buffer), and antibodies were used at a final concentration of 1:50. After 20 minutes incubation, cells were washed two times with 1 ml FACS Buffer and analyzed using a BD FACSCanto II. Data acquisition was made using DIVA software and analyzed using FlowJo 10.

### Apoptosis analysis using live-cell imaging

96-well plates were coated with 0,001 % Poly-L-Lysin (Merck #A-005-C). Cells were seeded at a density of 1 x 10⁴/well in RPMI without phenol red (GIBCO #11835105) containing 0,5 % BSA. Caspase 3/7 green apoptosis assay reagent (Sartorius #4440) was added to each well at a final concentration of 5 µM. Cells were stimulated with 2 mM H₂O₂ (Merck #H1009) or PBS as vehicle control. Cells were imaged in phase contrast and green fluorescence at 10x magnification every 30 minutes on an IncuCyte S3 Live cell analysis system.

### Live-cell measurement of phagocytosis

On day 14 of ex *vivo* differentiation, cells were seeded at a density of 10⁴/well in a 96-well plate in RPMI without phenol red containing 0.5 % BSA. 10 µg/well of green *E. coli* bioparticles (IncuCyte pHrodo, Essen Bioscience #4616) were added, according to manufacturer's recommendation. Each well's images were acquired every 30 minutes at 10x magnification in the green channel on an IncuCyte S3 Live cell analysis system. Images were analyzed using IncuCyte Basic Software according to the manufacturers' recommendations.

### Quantitative analysis of NETosis formation

*Ex vivo* differentiated cells were seeded at a density of 2 x 10⁴ per well in a Poly-L-Lysin coated 96-well plate. Cells were cultured in RPMI without phenol red supplemented with 0.5 % BSA and IncuCyte^{®} Cytotox Green Dye (Incucyte #4633) with a final concentration of 250 nM. NETosis was induced by adding 0.5 µM Phorbol-12-myristate-13-acetate (PMA). Cells were analyzed every 30 minutes at 20x magnification on the IncuCyte S3 Live cell analysis system. Data analysis was performed using IncuCyte Basic Software, as recommended by the manufacturer.

### ROS production after fMLP stimulation

Measurement of ROS upon stimulation with 50 nM N-Formylmethionine-leucyl-phenylalanine (fMLP) (Merck # 47729) was carried out according to the manufacturer's recommendations (Promega #G8820) and measured on a GloMax-Multidetection microplate reader (Promega).

### Statistical analysis

Statistical analysis was carried out by GraphPad Prism 7 software. A two-sided unpaired student's t-test was used to test for statistical significance if not indicated differently.

### 3. Results

### sgRNAs, primers and the repair template

**Table 1: Oligonucleotide sequences of sgRNAs targeting HAX1.* SEQ ID NOS: 1-553 are all on Chr1.**

| SEQ ID NO: | Position | Strand | Sequence | PAM | Specificity score | Efficiency score |
|---|---|---|---|---|---|---|
| 1 | 154272527 | -1 | AGGTCTTCCTCAAATTTGAA | AGG | 33,0632857 | 46,11521491 |
| 2 | 154272531 | 1 | GATAAAGCCTTTCAAATTTG | AGG | 29,3693276 | |
| 3 | 154272542 | 1 | TCAAATTTGAGGAAGACCTT | CGG | 35,3698332 | 56,6390463 |
| 4 | 154272547 | -1 | TGAAATGGAGGCGGGACCGA | AGG | 44,885456 | 59,8142246 |
| 5 | 154272555 | -1 | GCCGGACGTGAAATGGAGGC | GGG | 41,6617409 | 56,892138 |
| 6 | 154272556 | -1 | AGCCGGACGTGAAATGGAGG | CGG | 39,4045063 | 53,9437552 |
| 7 | 154272559 | -1 | GTAAGCCGGACGTGAAATGG | AGG | 46,6698277 | 69,2011281 |
| 8 | 154272562 | -1 | ACGGTAAGCCGGACGTGAAA | TGG | 48,9918062 | 35,0221683 |
| 9 | 154272565 | 1 | TCCCGCCTCCATTTCACGTC | CGG | 46,164549 | 43,5119352 |
| 10 | 154272573 | -1 | TGTCGTAAACGACGGTAAGC | CGG | 49,8088283 | 57,303012 |
| 11 | 154272581 | -1 | CCTGACACTGTCGTAAACGA | CGG | 48,3417929 | 70,394566 |
| 12 | 154272592 | 1 | CCGTCGTTTACGACAGTGTC | AGG | 48,9454976 | 47,9139372 |
| 13 | 154272599 | 1 | TTACGACAGTGTCAGGATCG | CGG | 46,0271259 | 61,9310541 |
| 14 | 154272600 | 1 | TACGACAGTGTCAGGATCGC | GGG | 47,4736666 | 53,2193739 |
| 15 | 154272612 | 1 | AGGATCGCGGGCTTGCTTTC | CGG | 45,1319716 | 26,386585 |
| 16 | 154272620 | 1 | GGGCTTGCTTTCCGGTAGCG | TGG | 48,6221135 | 54,0065297 |
| 17 | 154272620 | -1 | GAGGCGTCAGCCCACGCTAC | CGG | 48,126746 | 46,8168529 |
| 18 | 154272621 | 1 | GGCTTGCTTTCCGGTAGCGT | GGG | 48,9118444 | 48,833184 |
| 19 | 154272639 | -1 | GCCCTGTGAGAAATTGAGCG | AGG | 45,4964081 | 71,0797765 |
| 20 | 154272648 | 1 | CGCCTCGCTCAATTTCTCAC | AGG | 45,5216396 | 48,2251449 |
| 21 | 154272649 | 1 | GCCTCGCTCAATTTCTCACA | GGG | 42,3571626 | 59,5879404 |
| 22 | 154272658 | 1 | AATTTCTCACAGGGCTGCGC | AGG | 44,1950948 | 43,9098132 |
| 23 | 154272673 | -1 | AGTGGTCCATTCGCAGACGG | GGG | 47,8400964 | 68,0657601 |
| 24 | 154272674 | -1 | CAGTGGTCCATTCGCAGACG | GGG | 48,122724 | 55,1772541 |
| 25 | 154272675 | -1 | CCAGTGGTCCATTCGCAGAC | GGG | 39,9434051 | 50,6893623 |
| 26 | 154272676 | -1 | TCCAGTGGTCCATTCGCAGA | CGG | 31,8237872 | 46,9990465 |
| 27 | 154272678 | 1 | AGGTTTCCCCCGTCTGCGAA | TGG | 47,6083551 | 52,7758455 |
| 28 | 154272686 | 1 | CCCGTCTGCGAATGGACCAC | TGG | 45,8126601 | 45,8126601 |
| 29 | 154272689 | 1 | GTCTGCGAATGGACCACTGG | AGG | 31,4778654 | 58,4159712 |
| 30 | 154272690 | 1 | TCTGCGAATGGACCACTGGA | GGG | 30,5128824 | 52,496252 |
| 31 | 154272691 | 1 | CTGCGAATGGACCACTGGAG | GGG | 29,2072087 | 63,6831724 |
| 32 | 154272691 | -1 | GAACCTTTGAACCCCTCCAG | TGG | 28,3967229 | 69,344195 |
| 33 | 154272699 | 1 | GGACCACTGGAGGGGTTCAA | AGG | 43,7526064 | 48,6221631 |
| 34 | 154272716 | 1 | CAAAGGTTCGCGTCCCAGTA | CGG | 48,1862005 | 42,8823692 |
| 35 | 154272717 | 1 | AAAGGTTCGCGTCCCAGTAC | GGG | 48,1281249 | 50,9498425 |
| 36 | 154272718 | -1 | AAGAGGCTCATTCCCGTACT | GGG | 47,9484847 | 53,1331244 |
| 37 | 154272719 | -1 | AAAGAGGCTCATTCCCGTAC | TGG | 47,516593 | 48,827424 |
| 38 | 154272735 | -1 | AGCCCCGGAAGAGATCAAAG | AGG | 29,2725351 | 78,2514762 |
| 39 | 154272742 | 1 | TGAGCCTCTTTGATCTCTTC | CGG | 26,4062941 | 23,221593 |
| 40 | 154272743 | 1 | GAGCCTCTTTGATCTCTTCC | GGG | 26,5118379 | 36,2875246 |
| 41 | 154272744 | 1 | AGCCTCTTTGATCTCTTCCG | GGG | 28,4431051 | 58,6783921 |
| 42 | 154272750 | -1 | CAGGAAAGCCGAAAAAGCCC | CGG | 27,3064222 | 54,821905 |
| 43 | 154272753 | 1 | GATCTCTTCCGGGGCTTTTT | CGG | 29,3849195 | 10,2795282 |
| 44 | 154272762 | 1 | CGGGGCTTTTTCGGCTTTCC | TGG | 30,3419301 | 22,7738562 |
| 45 | 154272769 | 1 | TTTTCGGCTTTCCTGGACCT | CGG | 27,5407873 | 52,7894127 |
| 46 | 154272769 | -1 | TACTCTCACCTCCGAGGTCC | AGG | 43,3342466 | 47,6444344 |
| 47 | 154272772 | 1 | TCGGCTTTCCTGGACCTCGG | AGG | 42,8022038 | 64,0725012 |
| 48 | 154272775 | -1 | CGGACCTACTCTCACCTCCG | AGG | 42,4254129 | 70,6110959 |
| 49 | 154272782 | 1 | TGGACCTCGGAGGTGAGAGT | AGG | 34,309768 | 58,9417124 |
| 50 | 154272787 | 1 | CTCGGAGGTGAGAGTAGGTC | CGG | 42,3111414 | 49,8364474 |
| 51 | 154272792 | 1 | AGGTGAGAGTAGGTCCGGCT | CGG | 45,2441235 | 60,8633246 |
| 52 | 154272795 | -1 | ACCCCCACCCTTGTCCGAGC | CGG | 44,6139986 | 50,6970558 |
| 53 | 154272798 | 1 | GAGTAGGTCCGGCTCGGACA | AGG | 47,0120162 | 52,519231 |
| 54 | 154272799 | 1 | AGTAGGTCCGGCTCGGACAA | GGG | 48,916912 | 57,1529641 |
| 55 | 154272802 | 1 | AGGTCCGGCTCGGACAAGGG | TGG | 45,6670051 | 44,7779807 |
| 56 | 154272803 | 1 | GGTCCGGCTCGGACAAGGGT | GGG | 44,6306755 | 45,8659521 |
| 57 | 154272804 | 1 | GTCCGGCTCGGACAAGGGTG | GGG | 42,9478033 | 32,7683418 |
| 58 | 154272805 | 1 | TCCGGCTCGGACAAGGGTGG | GGG | 41,3293386 | 44,3219892 |
| 59 | 154272815 | 1 | ACAAGGGTGGGGGTCGTCTG | AGG | 44,3922135 | 37,4012446 |
| 60 | 154272816 | 1 | CAAGGGTGGGGGTCGTCTGA | GGG | 45,5316569 | 44,3778859 |
| 61 | 154272817 | 1 | AAGGGTGGGGGTCGTCTGAG | GGG | 42,2382935 | 45,7276653 |
| 62 | 154272836 | -1 | TTTCCAAAAATAAGACGTAG | GGG | 41,8301838 | 62,4532682 |
| 63 | 154272837 | -1 | TTTTCCAAAAATAAGACGTA | GGG | 38,6426811 | 50,7379353 |
| 64 | 154272838 | -1 | TTTTTCCAAAAATAAGACGT | AGG | 39,4497628 | 60,3188133 |
| 65 | 154272844 | 1 | TGACCCCTACGTCTTATTTT | TGG | 41,9893131 | 14,3074031 |
| 66 | 154272864 | -1 | GAGTTGAAGGAGTGGGACAG | AGG | 30,3070457 | 65,1607177 |
| 67 | 154272871 | -1 | TCTGCAGGAGTTGAAGGAGT | GGG | 31,709161 | 59,5212278 |
| 68 | 154272872 | -1 | CTCTGCAGGAGTTGAAGGAG | TGG | 32,471143 | 55,4416958 |
| 69 | 154272877 | -1 | GTCCTCTCTGCAGGAGTTGA | AGG | 27,3617476 | 34,0627551 |
| 70 | 154272886 | 1 | CTCCTTCAACTCCTGCAGAG | AGG | 33,8790723 | 70,4675181 |
| 71 | 154272886 | -1 | GCGACTTCTGTCCTCTCTGC | AGG | 31,738543 | 46,2565001 |
| 72 | 154272924 | -1 | CCGCCTTGGTTACTGTGGCG | GGG | 46,6565619 | 64,2075998 |
| 73 | 154272925 | -1 | ACCGCCTTGGTTACTGTGGC | GGG | 41,8007174 | 39,5782184 |
| 74 | 154272926 | -1 | GACCGCCTTGGTTACTGTGG | CGG | 43,0562456 | 54,7678321 |
| 75 | 154272929 | -1 | AATGACCGCCTTGGTTACTG | TGG | 44,5776138 | 53,0235433 |
| 76 | 154272932 | 1 | TCACCCCGCCACAGTAACCA | AGG | 45,1004815 | 65,5405466 |
| 77 | 154272935 | 1 | CCCCGCCACAGTAACCAAGG | CGG | 43,3889036 | 76,3998415 |
| 78 | 154272938 | -1 | TCTCCTCTTAATGACCGCCT | TGG | 46,725846 | 47,2779758 |
| 79 | 154272946 | 1 | TAACCAAGGCGGTCATTAAG | AGG | 45,5465567 | 49,1992697 |
| 80 | 154272975 | -1 | CATCACCCCAGGTTGGAGAA | AGG | 37,9328611 | 51,7900788 |
| 81 | 154272979 | 1 | AACTAAGCCTTTCTCCAACC | TGG | 38,2076364 | 45,0483703 |
| 82 | 154272980 | 1 | ACTAAGCCTTTCTCCAACCT | GGG | 37,328698 | 53,6610266 |
| 83 | 154272981 | 1 | CTAAGCCTTTCTCCAACCTG | GGG | 36,0411085 | 66,4188098 |
| 84 | 154272982 | -1 | CCTGTTGCATCACCCCAGGT | TGG | 39,9970632 | 54,9360039 |
| 85 | 154272986 | -1 | GGTCCCTGTTGCATCACCCC | AGG | 39,5248246 | 50,1631424 |
| 86 | 154272993 | 1 | CCAACCTGGGGTGATGCAAC | AGG | 44,3071177 | 39,8944864 |
| 87 | 154272994 | 1 | CAACCTGGGGTGATGCAACA | GGG | 39,9660727 | 68,3486159 |
| 88 | 154273000 | 1 | GGGGTGATGCAACAGGGACC | CGG | 37,3907235 | 52,7852641 |
| 89 | 154273001 | 1 | GGGTGATGCAACAGGGACCC | GGG | 36,5317595 | 56,4863145 |
| 90 | 154273004 | 1 | TGATGCAACAGGGACCCGGG | CGG | 42,3041658 | 55,588308 |
| 91 | 154273005 | 1 | GATGCAACAGGGACCCGGGC | GGG | 42,8908283 | 41,9417446 |
| 92 | 154273006 | 1 | ATGCAACAGGGACCCGGGCG | GGG | 45,7249093 | 44,391222 |
| 93 | 154273007 | -1 | CCTCACGGTCTTCCCCGCCC | GGG | 39,47598 | 56,4831958 |
| 94 | 154273008 | -1 | CCCTCACGGTCTTCCCCGCC | CGG | 42,126063 | 41,6607247 |
| 95 | 154273018 | 1 | CCCGGGCGGGGAAGACCGTG | AGG | 43,9926644 | 41,339228 |
| 96 | 154273019 | 1 | CCGGGCGGGGAAGACCGTGA | GGG | 46,1313431 | 66,9016285 |
| 97 | 154273022 | -1 | TCTTATTCCCCAGACCCTCA | CGG | 36,3794633 | 64,2844245 |
| 98 | 154273024 | 1 | CGGGGAAGACCGTGAGGGTC | TGG | 42,0412304 | 23,0499248 |
| 99 | 154273025 | 1 | GGGGAAGACCGTGAGGGTCT | GGG | 37,3704396 | 39,608163 |
| 100 | 154273026 | 1 | GGGAAGACCGTGAGGGTCTG | GGG | 2,6778798 | 48,0933188 |
| 101 | 154273052 | 1 | AAGACAGTGAGCAAGTGAGC | AGG | 32,4957596 | 57,8546459 |
| 102 | 154273059 | 1 | TGAGCAAGTGAGCAGGACCT | TGG | 34,318598 | 48,5717977 |
| 103 | 154273060 | 1 | GAGCAAGTGAGCAGGACCTT | GGG | 37,1130994 | 41,9883858 |
| 104 | 154273065 | -1 | AGTCCCTCCTCTCTCTCCCA | AGG | 26,7149289 | 54,1746006 |
| 105 | 154273069 | 1 | AGCAGGACCTTGGGAGAGAG | AGG | 26,8424511 | 54,5833841 |
| 106 | 154273072 | 1 | AGGACCTTGGGAGAGAGAGG | AGG | 24,9771148 | 63,2877325 |
| 107 | 154273073 | 1 | GGACCTTGGGAGAGAGAGGA | GGG | 25,8331438 | 61,6644917 |
| 108 | 154273078 | 1 | TTGGGAGAGAGAGGAGGGAC | TGG | 19,9880153 | 39,2071011 |
| 109 | 154273079 | 1 | TGGGAGAGAGAGGAGGGACT | GGG | 25,8355378 | 41,5493064 |
| 110 | 154273080 | 1 | GGGAGAGAGAGGAGGGACTG | GGG | 23,8839905 | 64,1740856 |
| 111 | 154273099 | 1 | GGGGCACACTGAAGAAAAAC | TGG | 33,2717277 | 28,1747379 |
| 112 | 154273100 | 1 | GGGCACACTGAAGAAAAACT | GGG | 30,3049101 | 56,4749877 |
| 113 | 154273101 | 1 | GGCACACTGAAGAAAAACTG | GGG | 31,897678 | 67,6384143 |
| 114 | 154273102 | 1 | GCACACTGAAGAAAAACTGG | GGG | 30,808447 | 72,7499858 |
| 115 | 154273106 | 1 | ACTGAAGAAAAACTGGGGGA | AGG | 30,8135967 | 62,0679636 |
| 116 | 154273116 | 1 | AACTGGGGGAAGGTGTATGA | AGG | 39,3546142 | 37,5449943 |
| 117 | 154273117 | 1 | ACTGGGGGAAGGTGTATGAA | GGG | 37,2152544 | 54,4769131 |
| 118 | 154273132 | 1 | ATGAAGGGAGCTGCGAGCTG | AGG | 39,3784232 | 61,2975863 |
| 119 | 154273188 | -1 | ACATCAAAACATTAATACAA | TGG | 28,3338487 | 51,276645 |
| 120 | 154273201 | 1 | ATTGTATTAATGTTTTGATG | TGG | 27,9376454 | 66,0940219 |
| 121 | 154273216 | -1 | TAGGGAGAGGGTCGGAGGAC | TGG | 38,7680778 | 41,2156896 |
| 122 | 154273221 | -1 | GAAGCTAGGGAGAGGGTCGG | AGG | 38,0982293 | 57,1755956 |
| 123 | 154273224 | -1 | TGGGAAGCTAGGGAGAGGGT | CGG | 25,6479749 | 67,4563151 |
| 124 | 154273228 | -1 | GGTCTGGGAAGCTAGGGAGA | GGG | 31,782752 | 69,1294183 |
| 125 | 154273229 | -1 | GGGTCTGGGAAGCTAGGGAG | AGG | 27,3005939 | 45,5817841 |
| 126 | 154273234 | -1 | GCAAGGGGTCTGGGAAGCTA | GGG | 34,2449814 | 46,5861956 |
| 127 | 154273235 | -1 | AGCAAGGGGTCTGGGAAGCT | AGG | 30,3671096 | 44,2183913 |
| 128 | 154273243 | -1 | GGGACAAGAGCAAGGGGTCT | GGG | 36,3279387 | 43,9975188 |
| 129 | 154273244 | -1 | TGGGACAAGAGCAAGGGGTC | TGG | 37,0495706 | 36,0325416 |
| 130 | 154273249 | -1 | CAAAGTGGGACAAGAGCAAG | GGG | 34,9668752 | 65,6230969 |
| 131 | 154273250 | -1 | GCAAAGTGGGACAAGAGCAA | GGG | 33,8390527 | 60,9486534 |
| 132 | 154273251 | -1 | GGCAAAGTGGGACAAGAGCA | AGG | 33,529974 | 50,535154 |
| 133 | 154273263 | -1 | CAACTCATGGGTGGCAAAGT | GGG | 40,3055615 | 46,8722368 |
| 134 | 154273264 | -1 | TCAACTCATGGGTGGCAAAG | TGG | 40,7151909 | 57,001189 |
| 135 | 154273272 | -1 | CCATTAAATCAACTCATGGG | TGG | 42,8265725 | 64,2203122 |
| 136 | 154273275 | -1 | AAGCCATTAAATCAACTCAT | GGG | 38,3716453 | 52,3257553 |
| 137 | 154273276 | -1 | TAAGCCATTAAATCAACTCA | TGG | 37,5502468 | 51,5284612 |
| 138 | 154273283 | 1 | CCACCCATGAGTTGATTTAA | TGG | 39,7635501 | 21,4807061 |
| 139 | 154273306 | 1 | CTTAAATAGTGCTGAAATAT | TGG | 33,7756849 | 29,2187649 |
| 140 | 154273309 | 1 | AAATAGTGCTGAAATATTGG | TGG | 32,9804558 | 56,3594095 |
| 141 | 154273321 | -1 | GGCTGAGAGTGGAGGCAGAT | TGG | 29,2335386 | 52,2981411 |
| 142 | 154273329 | -1 | ATCTCTGTGGCTGAGAGTGG | AGG | 30,0145458 | 57,8954431 |
| 143 | 154273332 | -1 | GGGATCTCTGTGGCTGAGAG | TGG | 30,5549904 | 55,5069827 |
| 144 | 154273342 | -1 | CTCCAAAAAAGGGATCTCTG | TGG | 27,0586383 | 70,7154439 |
| 145 | 154273351 | 1 | AGCCACAGAGATCCCTTTTT | TGG | 26,7259186 | 12,804478 |
| 146 | 154273352 | -1 | CGAGTCATCCCTCCAAAAAA | GGG | 29,8674468 | 42,1589987 |
| 147 | 154273353 | -1 | TCGAGTCATCCCTCCAAAAA | AGG | 28,2486801 | 37,7415893 |
| 148 | 154273354 | 1 | CACAGAGATCCCTTTTTGG | AGG | 27,7619225 | 54,4976635 |
| 149 | 154273355 | 1 | ACAGAGATCCCTTTTTTGGA | GGG | 26,8524792 | 58,3501763 |
| 150 | 154273383 | 1 | TCGAGATGAAGATGATGATG | AGG | 20,4544859 | 62,6189969 |
| 151 | 154273399 | 1 | GATGAGGAAGAAGAAGAAGA | AGG | 11,5629585 | 53,0122828 |
| 152 | 154273400 | 1 | ATGAGGAAGAAGAAGAAGAA | GGG | 14,298029 | 51,7057323 |
| 153 | 154273401 | 1 | TGAGGAAGAAGAAGAAGAAG | GGG | 9,0465464 | 59,886835 |
| 154 | 154273402 | 1 | GAGGAAGAAGAAGAAGAAGG | GGG | 13,7475138 | 70,3133863 |
| 155 | 154273409 | 1 | AAGAAGAAGAAGGGGGCTCA | TGG | 31,0538775 | 44,4863567 |
| 156 | 154273410 | 1 | AGAAGAAGAAGGGGGCTCAT | GGG | 35,1263938 | 49,4828161 |
| 157 | 154273411 | 1 | GAAGAAGAAGGGGGCTCATG | GGG | 35,2495598 | 62,4659578 |
| 158 | 154273417 | 1 | GAAGGGGGCTCATGGGGCCG | TGG | 34,2574291 | 24,1088161 |
| 159 | 154273418 | 1 | AAGGGGGCTCATGGGGCCGT | GGG | 42,1401196 | 53,3822417 |
| 160 | 154273423 | -1 | TATGGAACCTTGGGTTCCCA | CGG | 27,4855463 | 64,6615096 |
| 161 | 154273427 | 1 | CATGGGGCCGTGGGAACCCA | AGG | 27,8832727 | 45,8479815 |
| 162 | 154273432 | -1 | GCTGAGGACTATGGAACCTT | GGG | 39,8536839 | 49,1641412 |
| 163 | 154273433 | -1 | TGCTGAGGACTATGGAACCT | TGG | 39,1457136 | 49,4909117 |
| 164 | 154273441 | -1 | CAGGGGGGTGCTGAGGACTA | TGG | 34,7634907 | 38,4497648 |
| 165 | 154273448 | -1 | AATTCCTCAGGGGGGTGCTG | AGG | 38,5417764 | 58,6386282 |
| 166 | 154273455 | 1 | TAGTCCTCAGCACCCCCCTG | AGG | 40,5462801 | 72,3474987 |
| 167 | 154273456 | -1 | CGAAGCCAAATTCCTCAGGG | GGG | 39,4577276 | 75,6820857 |
| 168 | 154273457 | -1 | CCGAAGCCAAATTCCTCAGG | GGG | 40,220298 | 65,1640099 |
| 169 | 154273458 | -1 | GCCGAAGCCAAATTCCTCAG | GGG | 39,5171419 | 67,1483159 |
| 170 | 154273459 | -1 | AGCCGAAGCCAAATTCCTCA | GGG | 41,411012 | 59,8439911 |
| 171 | 154273460 | -1 | AAGCCGAAGCCAAATTCCTC | AGG | 40,7634692 | 50,6818401 |
| 172 | 154273462 | 1 | CAGCACCCCCCTGAGGAATT | TGG | 40,6023978 | 36,7741791 |
| 173 | 154273468 | 1 | CCCCCTGAGGAATTTGGCTT | CGG | 35,4072841 | 37,7815583 |
| 174 | 154273486 | 1 | TTCGGCTTCAGCTTCAGCCC | AGG | 32,9797797 | 50,3273739 |
| 175 | 154273489 | 1 | GGCTTCAGCTTCAGCCCAGG | AGG | 22,616862 | 60,9420785 |
| 176 | 154273492 | 1 | TTCAGCTTCAGCCCAGGAGG | AGG | 20,106228 | 51,7263324 |
| 177 | 154273492 | -1 | GGAAACGTATCCCTCCTCCT | GGG | 29,5226941 | 59,6937846 |
| 178 | 154273493 | 1 | TCAGCTTCAGCCCAGGAGGA | GGG | 20,1889084 | 66,3809282 |
| 179 | 154273493 | -1 | TGGAAACGTATCCCTCCTCC | TGG | 29,1759 | 47,6107056 |
| 180 | 154273513 | -1 | CATCAAAGCCGAAGTTATCG | TGG | 32,380861 | 69,0591175 |
| 181 | 154273516 | 1 | ATACGTTTCCACGATAACTT | CGG | 31,9223552 | 56,6435013 |
| 182 | 154273537 | -1 | TGCTATTGAAATCTCGTACT | AGG | 41,6711554 | 55,9954947 |
| 183 | 154273563 | 1 | CAATAGCATCTTCAGCGATA | TGG | 39,3038034 | 40,5608097 |
| 184 | 154273564 | 1 | AATAGCATCTTCAGCGATAT | GGG | 43,3991993 | 39,5947239 |
| 185 | 154273565 | 1 | ATAGCATCTTCAGCGATATG | GGG | 41,3137551 | 58,6824924 |
| 186 | 154273566 | 1 | TAGCATCTTCAGCGATATGG | GGG | 44,4169197 | 59,8475294 |
| 187 | 154273571 | 1 | TCTTCAGCGATATGGGGGCC | TGG | 34,8589477 | 48,8976065 |
| 188 | 154273578 | -1 | AGGATGGGAAGGCAAGGTCC | AGG | 25,6391491 | 55,0506934 |
| 189 | 154273584 | -1 | ACCAGGAGGATGGGAAGGCA | AGG | 23,8008494 | 60,4246644 |
| 190 | 154273589 | -1 | CACACACCAGGAGGATGGGA | AGG | 31,6614459 | 60,5183264 |
| 191 | 154273593 | -1 | AAGCCACACACCAGGAGGAT | GGG | 35,8553368 | 52,3524408 |
| 192 | 154273594 | 1 | ACCTTGCCTTCCCATCCTCC | TGG | 29,1021078 | 49,440897 |
| 193 | 154273594 | -1 | AAAGCCACACACCAGGAGGA | TGG | 27,3283287 | 47,5737798 |
| 194 | 154273598 | -1 | AGGGAAAGCCACACACCAGG | AGG | 34,9942733 | 69,8702402 |
| 195 | 154273601 | 1 | CTTCCCATCCTCCTGGTGTG | TGG | 32,7916568 | 58,7046758 |
| 196 | 154273601 | -1 | CTTAGGGAAAGCCACACACC | AGG | 30,2146524 | 62,110824 |
| 197 | 154273613 | 1 | CTGGTGTGTGGCTTTCCCTA | AGG | 36,3987315 | 43,268137 |
| 198 | 154273614 | 1 | TGGTGTGTGGCTTTCCCTAA | GGG | 39,1730219 | 41,0457797 |
| 199 | 154273615 | 1 | GGTGTGTGGCTTTCCCTAAG | GGG | 40,1479078 | 53,5699978 |
| 200 | 154273617 | -1 | AAACCACAGGTTGCCCCTTA | GGG | 43,1287174 | 42,7998137 |
| 201 | 154273618 | -1 | GAAACCACAGGTTGCCCCTT | AGG | 41,1780488 | 44,400966 |
| 202 | 154273625 | 1 | TTTCCCTAAGGGGCAACCTG | TGG | 40,141488 | 56,366284 |
| 203 | 154273630 | -1 | ACCAACCCACCAGAAACCAC | AGG | 37,3163715 | 64,7326908 |
| 204 | 154273632 | 1 | AAGGGGCAACCTGTGGTTTC | TGG | 39,0009329 | 19,4531889 |
| 205 | 154273635 | 1 | GGGCAACCTGTGGTTTCTGG | TGG | 35,910398 | 49,5548189 |
| 206 | 154273636 | 1 | GGCAACCTGTGGTTTCTGGT | GGG | 36,8390544 | 54,2840464 |
| 207 | 154273640 | 1 | ACCTGTGGTTTCTGGTGGGT | TGG | 36,192591 | 41,119323 |
| 208 | 154273643 | 1 | TGTGGTTTCTGGTGGGTTGG | TGG | 34,3756221 | 37,6891623 |
| 209 | 154273644 | 1 | GTGGTTTCTGGTGGGTTGGT | GGG | 36,6723262 | 45,0322534 |
| 210 | 154273664 | 1 | GGGTGAAATAAAGAGCCTGC | AGG | 37,5826341 | 53,0807481 |
| 211 | 154273665 | 1 | GGTGAAATAAAGAGCCTGCA | GGG | 35,5357215 | 62,7662733 |
| 212 | 154273668 | -1 | ATCCCCCAGCTACTCCCTGC | AGG | 34,3016556 | 44,2068803 |
| 213 | 154273674 | 1 | AAGAGCCTGCAGGGAGTAGC | TGG | 25,0825758 | 33,6214058 |
| 214 | 154273675 | 1 | AGAGCCTGCAGGGAGTAGCT | GGG | 37,6850255 | 44,9680501 |
| 215 | 154273676 | 1 | GAGCCTGCAGGGAGTAGCTG | GGG | 34,4852398 | 57,3283169 |
| 216 | 154273677 | 1 | AGCCTGCAGGGAGTAGCTGG | GGG | 34,7897719 | 65,7039205 |
| 217 | 154273681 | 1 | TGCAGGGAGTAGCTGGGGGA | TGG | 26,6126286 | 40,9230495 |
| 218 | 154273682 | 1 | GCAGGGAGTAGCTGGGGGAT | GGG | 30,5829522 | 45,9248467 |
| 219 | 154273734 | -1 | GCTGGGATGGGAAAGGACTT | GGG | 33,5983029 | 48,6679898 |
| 220 | 154273735 | -1 | TGCTGGGATGGGAAAGGACT | TGG | 31,6975098 | 25,9795305 |
| 221 | 154273741 | -1 | GGTGTTTGCTGGGATGGGAA | AGG | 28,6213721 | 55,4643005 |
| 222 | 154273746 | -1 | TGGCAGGTGTTTGCTGGGAT | GGG | 33,7505734 | 54,6686288 |
| 223 | 154273747 | -1 | GTGGCAGGTGTTTGCTGGGA | TGG | 30,7876355 | 42,0280742 |
| 224 | 154273751 | -1 | AAAGGTGGCAGGTGTTTGCT | GGG | 30,3489536 | 59,3473817 |
| 225 | 154273752 | -1 | GAAAGGTGGCAGGTGTTTGC | TGG | 37,5518054 | 40,849317 |
| 226 | 154273762 | -1 | AAGTTCTGCAGAAAGGTGGC | AGG | 34,8382734 | 55,076261 |
| 227 | 154273766 | -1 | CTGGAAGTTCTGCAGAAAGG | TGG | 30,7174647 | 62,5490497 |
| 228 | 154273769 | -1 | GACCTGGAAGTTCTGCAGAA | AGG | 33,0389832 | 63,59257 |
| 229 | 154273778 | 1 | CACCTTTCTGCAGAACTTCC | AGG | 35,616152 | 31,65377 |
| 230 | 154273785 | -1 | GGTGTCTCTGACTCAGGACC | TGG | 32,613505 | 59,8059067 |
| 231 | 154273791 | -1 | TCACCAGGTGTCTCTGACTC | AGG | 35,5485997 | 55,6340924 |
| 232 | 154273799 | 1 | GGTCCTGAGTCAGAGACACC | TGG | 26,6601324 | 61,2514576 |
| 233 | 154273806 | -1 | CCCTCCCGTAGTCTCTCACC | AGG | 32,3516226 | 56,2549958 |
| 234 | 154273812 | 1 | AGACACCTGGTGAGAGACTA | CGG | 34,1632423 | 40,0030562 |
| 235 | 154273813 | 1 | GACACCTGGTGAGAGACTAC | GGG | 38,8895696 | 60,6525972 |
| 236 | 154273816 | 1 | ACCTGGTGAGAGACTACGGG | AGG | 42,2810953 | 67,114313 |
| 237 | 154273817 | 1 | CCTGGTGAGAGACTACGGGA | GGG | 40,5848768 | 72,3426355 |
| 238 | 154273830 | 1 | TACGGGAGGGACAGACACTT | CGG | 39,5487952 | 54,5704823 |
| 239 | 154273831 | 1 | ACGGGAGGGACAGACACTTC | GGG | 38,2089877 | 47,7910313 |
| 240 | 154273860 | -1 | ATCCTGGGCTGGTGACTATC | TGG | 30,9735134 | 35,4413091 |
| 241 | 154273869 | 1 | ATCCAGATAGTCACCAGCCC | AGG | 29,0158474 | 56,1200087 |
| 242 | 154273871 | -1 | CCCCCCCAAAGATCCTGGGC | TGG | 30,832318 | 54,9426691 |
| 243 | 154273875 | -1 | AAGACCCCCCCAAAGATCCT | GGG | 29,8253374 | 55,0468828 |
| 244 | 154273876 | -1 | CAAGACCCCCCCAAAGATCC | TGG | 34,1644815 | 40,2579282 |
| 245 | 154273877 | 1 | AGTCACCAGCCCAGGATCTT | TGG | 38,8450915 | 33,2111975 |
| 246 | 154273878 | 1 | GTCACCAGCCCAGGATCTTT | GGG | 28,6067566 | 21,6488737 |
| 247 | 154273879 | 1 | TCACCAGCCCAGGATCTTTG | GGG | 27,9904016 | 58,594611 |
| 248 | 154273880 | 1 | CACCAGCCCAGGATCTTTGG | GGG | 35,5083348 | 59,8090317 |
| 249 | 154273881 | 1 | ACCAGCCCAGGATCTTTGGG | GGG | 35,7151977 | 59,8221261 |
| 250 | 154273882 | 1 | CCAGCCCAGGATCTTTGGGG | GGG | 35,8798933 | 64,520438 |
| 251 | 154273888 | 1 | CAGGATCTTTGGGGGGGTCT | TGG | 34,2689921 | 34,8315123 |
| 252 | 154273921 | -1 | CCAGTCTGGTGCTGGTTGGG | GGG | 35,7750333 | 58,0253248 |
| 253 | 154273922 | -1 | CCCAGTCTGGTGCTGGTTGG | GGG | 35,5009049 | 42,2774344 |
| 254 | 154273923 | -1 | CCCCAGTCTGGTGCTGGTTG | GGG | 39,9329118 | 37,0679744 |
| 255 | 154273924 | -1 | GCCCCAGTCTGGTGCTGGTT | GGG | 40,4729009 | 21,9794679 |
| 256 | 154273925 | -1 | AGCCCCAGTCTGGTGCTGGT | TGG | 38,9596118 | 38,3820558 |
| 257 | 154273929 | -1 | TGGGAGCCCCAGTCTGGTGC | TGG | 35,9271534 | 46,5842372 |
| 258 | 154273932 | 1 | CCCCCCAACCAGCACCAGAC | TGG | 38,3292839 | 44,2142708 |
| 259 | 154273933 | 1 | CCCCCAACCAGCACCAGACT | GGG | 37,0463847 | 44,4417255 |
| 260 | 154273934 | 1 | CCCCAACCAGCACCAGACTG | GGG | 36,0420448 | 68,1434322 |
| 261 | 154273935 | -1 | GGCCTCTGGGAGCCCCAGTC | TGG | 25,0220779 | 44,0422481 |
| 262 | 154273944 | 1 | CACCAGACTGGGGCTCCCAG | AGG | 30,312565 | 69,828566 |
| 263 | 154273948 | -1 | CACCCTATGAAATGGCCTCT | GGG | 38,8374092 | 53,3044805 |
| 264 | 154273949 | -1 | TCACCCTATGAAATGGCCTC | TGG | 41,904154 | 48,261122 |
| 265 | 154273956 | 1 | GCTCCCAGAGGCCATTTCAT | AGG | 31,8447355 | 46,1025552 |
| 266 | 154273956 | -1 | GGGATACTCACCCTATGAAA | TGG | 43,6924768 | 51,7924739 |
| 267 | 154273957 | 1 | CTCCCAGAGGCCATTTCATA | GGG | 37,634212 | 45,1928084 |
| 268 | 154273973 | 1 | CATAGGGTGAGTATCCCATC | TGG | 45,2559759 | 56,3070909 |
| 269 | 154273976 | -1 | CTCTCACTTCAGGACCAGAT | GGG | 41,5330984 | 56,3894291 |
| 270 | 154273977 | -1 | GCTCTCACTTCAGGACCAGA | TGG | 35,1539197 | 53,2748055 |
| 271 | 154273986 | -1 | CTCTCACAAGCTCTCACTTC | AGG | 38,6088619 | 35,727296 |
| 272 | 154274010 | -1 | cAGTCTTTGCACTTTATTAG | TGG | 56,4685543 | 37,5113766 |
| 273 | 154274022 | 1 | CACTAATAAAGTGCAAAGAC | Tgg | 59,8767253 | 54,9089496 |
| 274 | 154274027 | 1 | ATAAAGTGCAAAGACTggct | agg | 57,0728966 | 59,3893994 |
| 275 | 154274032 | 1 | GTGCAAAGACTggctaggtg | cgg | 75,715051 | 64,7154321 |
| 276 | 154274035 | 1 | CAAAGACTggctaggtgcgg | tgg | 83,9355993 | 54,2173375 |
| 277 | 154274053 | -1 | tcccaaagtgctgggattac | agg | 15,4572314 | 31,8499203 |
| 278 | 154274061 | -1 | cctcggcctcccaaagtgct | ggg | 12,0649158 | 49,8956636 |
| 279 | 154274062 | 1 | cgcctgtaatcccagcactt | tgg | 18,1361646 | 48,7859724 |
| 280 | 154274062 | -1 | acctcggcctcccaaagtgc | tgg | 19,9980021 | 35,074852 |
| 281 | 154274063 | 1 | gcctgtaatcccagcacttt | ggg | 10,9293449 | 26,6323568 |
| 282 | 154274066 | 1 | tgtaatcccagcactttggg | agg | 14,3621285 | 62,399855 |
| 283 | 154274072 | 1 | cccagcactttgggaggccg | agg | 18,7122237 | 46,4517538 |
| 284 | 154274075 | 1 | agcactttgggaggccgagg | tgg | 27,1692661 | 49,5695628 |
| 285 | 154274076 | 1 | gcactttgggaggccgaggt | ggg | 12,3993754 | 57,0082778 |
| 286 | 154274078 | -1 | ctctggtgatctgcccacct | cgg | 43,4432621 | 61,6729102 |
| 287 | 154274090 | 1 | cgaggtgggcagatcaccag | agg | 68,5936854 | 63,4809908 |
| 288 | 154274095 | 1 | tgggcagatcaccagaggtc | agg | 63,0770062 | 46,9586225 |
| 289 | 154274095 | -1 | ggtctccaactcctgacctc | tgg | 56,8127476 | 47,1014386 |
| 290 | 154274101 | 1 | gatcaccagaggtcaggagt | tgg | 49,8896237 | 55,3311912 |
| 291 | 154274113 | 1 | tcaggagttggagaccagcc | tgg | 44,8605887 | 57,5270497 |
| 292 | 154274116 | -1 | tttcaccatgttgaccaggc | tgg | 64,3247792 | 45,1226512 |
| 293 | 154274120 | -1 | ggggtttcaccatgttgacc | agg | 62,8220971 | 55,7859264 |
| 294 | 154274122 | 1 | ggagaccagcctggtcaaca | tgg | 22,6962032 | 62,2337368 |
| 295 | 154274139 | -1 | ttgtatttttagtagacacg | ggg | 31,9945861 | 67,8696087 |
| 296 | 154274140 | -1 | tttgtatttttagtagacac | ggg | 32,7591805 | 52,2822211 |
| 297 | 154274141 | -1 | ttttgtatttttagtagaca | cgg | 17,3717037 | 62,7421821 |
| 298 | 154274162 | 1 | taaaaatacaaaaaattagc | cgg | 33,501409 | 46,3520595 |
| 299 | 154274163 | 1 | aaaaatacaaaaaattagcc | ggg | 39,2431802 | 58,1969387 |
| 300 | 154274168 | 1 | tacaaaaaattagccgggca | tgg | 71,6942599 | 51,3153298 |
| 301 | 154274170 | -1 | caggcacctgccaccatgcc | cgg | 21,0746074 | 47,9337804 |
| 302 | 154274171 | 1 | aaaaaattagccgggcatgg | tgg | 51,1753365 | 65,6288275 |
| 303 | 154274175 | 1 | aattagccgggcatggtggc | agg | 39,9667894 | 43,1766528 |
| 304 | 154274189 | -1 | tcctgagtagctggcattac | agg | 71,2404551 | 29,0525621 |
| 305 | 154274198 | -1 | gcctcagcctcctgagtagc | tgg | 44,2954798 | 36,5340539 |
| 306 | 154274199 | 1 | gcctgtaatgccagctactc | agg | 38,4504167 | 37,9449621 |
| 307 | 154274202 | 1 | tgtaatgccagctactcagg | agg | 56,1311676 | 68,3427471 |
| 308 | 154274208 | 1 | gccagctactcaggaggctg | agg | 24,752803 | 49,0119429 |
| 309 | 154274212 | 1 | gctactcaggaggctgaggc | agg | 15,0924097 | 45,7219912 |
| 310 | 154274220 | 1 | ggaggctgaggcaggagaat | tgg | 38,8494451 | 37,9510198 |
| 311 | 154274231 | 1 | caggagaattggttgaaccc | agg | 76,7357348 | 62,3971695 |
| 312 | 154274234 | 1 | gagaattggttgaacccagg | agg | 65,603322 | 70,2300571 |
| 313 | 154274237 | 1 | aattggttgaacccaggagg | tgg | 56,553259 | 53,4057308 |
| 314 | 154274237 | -1 | AGATGGAGTctccacctcct | ggg | 59,5736274 | 48,5898898 |
| 315 | 154274238 | -1 | GAGATGGAGTctccacctcc | tgg | 54,2188951 | 34,9371248 |
| 316 | 154274254 | -1 | gtctcaaacaaTTTTTGAGA | TGG | 47,9321613 | 46,284192 |
| 317 | 154274276 | -1 | ctgggcaacagagcaaggct | ggg | 31,1723416 | 57,0433765 |
| 318 | 154274277 | -1 | cctgggcaacagagcaaggc | tgg | 45,9037479 | 40,3632303 |
| 319 | 154274281 | -1 | acagcctgggcaacagagca | agg | 48,1304256 | 55,8404093 |
| 320 | 154274288 | 1 | ccagccttgctctgttgccc | agg | 41,9855968 | 40,9682413 |
| 321 | 154274294 | -1 | gtgccacggcactacagcct | ggg | 78,5547877 | 54,0836492 |
| 322 | 154274295 | -1 | tgtgccacggcactacagcc | tgg | 76,7010836 | 45,3573536 |
| 323 | 154274302 | 1 | ttgcccaggctgtagtgccg | tgg | 72,617414 | 61,1314712 |
| 324 | 154274308 | -1 | agtgagctgagattgtgcca | cgg | 46,1454472 | 79,2872839 |
| 325 | 154274335 | -1 | cacttgaacctgggaattgg | agg | 58,7520471 | 38,7494258 |
| 326 | 154274338 | 1 | cactgcaacctccaattccc | agg | 62,706228 | 53,7026777 |
| 327 | 154274338 | -1 | aatcacttgaacctgggaat | tgg | 37,4274858 | 35,8354252 |
| 328 | 154274344 | -1 | caggagaatcacttgaacct | ggg | 34,3345883 | 58,9649258 |
| 329 | 154274345 | -1 | gcaggagaatcacttgaacc | tgg | 23,8016875 | 48,9946706 |
| 330 | 154274363 | -1 | gctactcaggaggctgaggc | agg | 15,0924097 | 47,8595628 |
| 331 | 154274367 | -1 | cccagctactcaggaggctg | agg | 27,5691022 | 47,8193259 |
| 332 | 154274373 | -1 | tgtaatcccagctactcagg | agg | 40,4458679 | 70,1123311 |
| 333 | 154274376 | -1 | gactgtaatcccagctactc | agg | 35,0946415 | 41,9775131 |
| 334 | 154274377 | 1 | gcctcagcctcctgagtagc | tgg | 44,2954798 | 28,2836774 |
| 335 | 154274378 | 1 | cctcagcctcctgagtagct | ggg | 26,0998417 | 48,273075 |
| 336 | 154274400 | -1 | aaattagctgggcgtttggc | agg | 83,8315913 | 44,740309 |
| 337 | 154274404 | -1 | acaaaaattagctgggcgtt | tgg | 78,9395789 | 38,2667152 |
| 338 | 154274411 | -1 | taaaaacacaaaaattagct | ggg | 31,2029058 | 53,8201862 |
| 339 | 154274412 | -1 | ctaaaaacacaaaaattagc | tgg | 22,5211049 | 41,6015847 |
| 340 | 154274432 | 1 | ttttgtgtttttagtagaga | tgg | 9,8843117 | 46,1154185 |
| 341 | 154274448 | 1 | gagatggcgtttcaccgtgt | tgg | 92,1896307 | 53,4183924 |
| 342 | 154274451 | -1 | caaaagcagcctggccaaca | cgg | 58,7908174 | 59,999641 |
| 343 | 154274453 | 1 | ggcgtttcaccgtgttggcc | agg | 79,1811791 | 40,9742056 |
| 344 | 154274460 | -1 | tcaggagctcaaaagcagcc | tgg | 49,0242541 | 55,1972908 |
| 345 | 154274478 | 1 | gcttttgagctcctgacctc | agg | 60,6983219 | 44,2003464 |
| 346 | 154274478 | -1 | tgggtggatcacctgaggtc | agg | 26,4805816 | 52,2412875 |
| 347 | 154274483 | -1 | cgaggtgggtggatcacctg | agg | 68,1107301 | 64,9091461 |
| 348 | 154274494 | -1 | ctttgggaggccgaggtggg | tgg | 27,5504106 | 44,4805294 |
| 349 | 154274495 | 1 | ctcaggtgatccacccacct | cgg | 42,1380398 | 66,8283846 |
| 350 | 154274497 | -1 | gcactttgggaggccgaggt | ggg | 12,3993754 | 54,0918706 |
| 351 | 154274498 | -1 | agcactttgggaggccgagg | tgg | 27,1692661 | 47,3591139 |
| 352 | 154274501 | -1 | tccagcactttgggaggccg | agg | 31,1851409 | 47,8491955 |
| 353 | 154274507 | -1 | tgtaattccagcactttggg | agg | 24,5767375 | 63,0043992 |
| 354 | 154274510 | -1 | acttgtaattccagcacttt | ggg | 43,221163 | 31,0839162 |
| 355 | 154274511 | 1 | acctcggcctcccaaagtgc | tgg | 19,9980021 | 44,0964606 |
| 356 | 154274511 | -1 | cacttgtaattccagcactt | tgg | 58,4099949 | 44,6018623 |
| 357 | 154274538 | -1 | CAATCTTTggccaggcatga | tgg | 64,3024855 | 50,7923107 |
| 358 | 154274539 | 1 | caagtgtgagccatcatgcc | tgg | 45,1548598 | 51,2901588 |
| 359 | 154274546 | -1 | AAGAATTGCAATCTTTggcc | agg | 63,6112836 | 46,7678824 |
| 360 | 154274551 | -1 | CAAACAAGAATTGCAATCTT | Tgg | 59,4815664 | 30,2942604 |
| 361 | 154274578 | 1 | TTGTTTGAATCTGATAGCCT | TGG | 40,7516847 | 47,263331 |
| 362 | 154274579 | 1 | TGTTTGAATCTGATAGCCTT | GGG | 39,770592 | 41,8160468 |
| 363 | 154274583 | 1 | TGAATCTGATAGCCTTGGGT | TGG | 40,1767696 | 58,4633506 |
| 364 | 154274584 | -1 | GGGCTTAGGATTCCAACCCA | AGG | 43,4376867 | 68,60457 |
| 365 | 154274598 | -1 | AAACACTAAATGGAGGGCTT | AGG | 39,7285843 | 42,9595764 |
| 366 | 154274604 | -1 | AACAGAAAACACTAAATGGA | GGG | 29,4676934 | 62,6381481 |
| 367 | 154274605 | -1 | AAACAGAAAACACTAAATGG | AGG | 31,3140462 | 65,9730394 |
| 368 | 154274608 | -1 | AGAAAACAGAAAACACTAAA | TGG | 20,9147787 | 34,150728 |
| 369 | 154274634 | 1 | TGTTTTCTATATTAAGCTTG | TGG | 35,9646886 | 41,724489 |
| 370 | 154274635 | 1 | GTTTTCTATATTAAGCTTGT | GGG | 36,6451144 | 34,3795655 |
| 371 | 154274646 | 1 | TAAGCTTGTGGGATTTTCTT | TGG | 23,5962538 | 16,5307738 |
| 372 | 154274647 | 1 | AAGCTTGTGGGATTTTCTTT | GGG | 30,3721129 | 23,3642816 |
| 373 | 154274648 | 1 | AGCTTGTGGGATTTTCTTTG | GGG | 31,875104 | 50,4186232 |
| 374 | 154274649 | 1 | GCTTGTGGGATTTTCTTTGG | GGG | 31,4332487 | 50,5153008 |
| 375 | 154274650 | 1 | CTTGTGGGATTTTCTTTGGG | GGG | 32,7162526 | 53,6436415 |
| 376 | 154274656 | 1 | GGATTTTCTTTGGGGGGAGA | TGG | 31,3469394 | 30,5342151 |
| 377 | 154274657 | 1 | GATTTTCTTTGGGGGGAGAT | GGG | 36,7872945 | 35,4336094 |
| 378 | 154274701 | 1 | caaaaacaaaaaaaCACCAC | TGG | 41,2159208 | 61,2981903 |
| 379 | 154274706 | -1 | CCAAGAATTATCTAGACCAG | TGG | 37,6094808 | 66,0262424 |
| 380 | 154274717 | 1 | CCACTGGTCTAGATAATTCT | TGG | 39,8102601 | 38,793539 |
| 381 | 154274723 | 1 | GTCTAGATAATTCTTGGATA | AGG | 37,2455616 | 41,4658241 |
| 382 | 154274727 | 1 | AGATAATTCTTGGATAAGGC | AGG | 37,8546739 | 53,7346159 |
| 383 | 154274741 | 1 | TAAGGCAGGATTGTGCACAG | AGG | 38,5180553 | 78,4410279 |
| 384 | 154274746 | 1 | CAGGATTGTGCACAGAGGAT | AGG | 36,6479203 | 50,920771 |
| 385 | 154274762 | 1 | GGATAGGAATATATCAGTTC | AGG | 39,034352 | 38,0282295 |
| 386 | 154274774 | 1 | ATCAGTTCAGGAAGTCTTTC | TGG | 34,9892748 | 20,6000212 |
| 387 | 154274781 | 1 | CAGGAAGTCTTTCTGGTAGA | AGG | 35,2732224 | 41,7929836 |
| 388 | 154274799 | 1 | GAAGGATACATAAAACAGCC | AGG | 37,8540709 | 58,3694536 |
| 389 | 154274803 | 1 | GATACATAAAACAGCCAGGA | AGG | 34,062795 | 60,2800857 |
| 390 | 154274806 | -1 | CTTATTTACACACTCCTTCC | TGG | 37,3403533 | 31,8115061 |
| 391 | 154274818 | 1 | CAGGAAGGAGTGTGTAAATA | AGG | 37,2733164 | 35,0264547 |
| 392 | 154274831 | 1 | GTAAATAAGGCTATTCTGAA | TGG | 37,2706963 | 52,6047233 |
| 393 | 154274848 | 1 | GAATGGAATTATCTCTTCTG | TGG | 32,9196203 | 53,6976004 |
| 394 | 154274852 | 1 | GGAATTATCTCTTCTGTGGA | AGG | 26,1196362 | 49,7527102 |
| 395 | 154274853 | 1 | GAATTATCTCTTCTGTGGAA | GGG | 31,5778285 | 54,0389602 |
| 396 | 154274854 | 1 | AATTATCTCTTCTGTGGAAG | GGG | 32,004909 | 50,1961962 |
| 397 | 154274855 | 1 | ATTATCTCTTCTGTGGAAGG | GGG | 30,3181326 | 54,9334637 |
| 398 | 154274861 | 1 | TCTTCTGTGGAAGGGGGTTT | TGG | 36,0387836 | 18,3355338 |
| 399 | 154274868 | 1 | TGGAAGGGGGTTTTGGAGCT | CGG | 33,1678094 | 37,2030208 |
| 400 | 154274869 | 1 | GGAAGGGGGTTTTGGAGCTC | GGG | 35,3275481 | 44,074224 |
| 401 | 154274881 | 1 | TGGAGCTCGGGAGTAGTTTG | AGG | 44,6752366 | 42,3123622 |
| 402 | 154274900 | -1 | CTTCCAATCTGAAATTTGAA | AGG | 30,3130034 | 43,9725483 |
| 403 | 154274908 | 1 | TGACCTTTCAAATTTCAGAT | TGG | 32,5985831 | 47,0796185 |
| 404 | 154274912 | 1 | CTTTCAAATTTCAGATTGGA | AGG | 35,8247613 | 51,0446633 |
| 405 | 154274932 | 1 | AGGAGTCTTTTCACTTACTA | TGG | 38,4476172 | 37,2929484 |
| 406 | 154274959 | 1 | TTCTGCAGTTTGATGATGTA | TGG | 38,4916061 | 42,160919 |
| 407 | 154274966 | 1 | GTTTGATGATGTATGGCCTA | TGG | 43,9532119 | 56,6808873 |
| 408 | 154274971 | -1 | GTTCTAGGATGGGGGTCCAT | AGG | 37,6484934 | 48,0235675 |
| 409 | 154274979 | -1 | CCTCTCTGGTTCTAGGATGG | GGG | 26,9826516 | 68,4886378 |
| 410 | 154274980 | -1 | TCCTCTCTGGTTCTAGGATG | GGG | 26,4701646 | 41,2575448 |
| 411 | 154274981 | -1 | GTCCTCTCTGGTTCTAGGAT | GGG | 28,2953037 | 41,0306258 |
| 412 | 154274982 | -1 | TGTCCTCTCTGGTTCTAGGA | TGG | 27,3360236 | 39,8862814 |
| 413 | 154274986 | -1 | CCATTGTCCTCTCTGGTTCT | AGG | 25,372498 | 30,8208222 |
| 414 | 154274990 | 1 | CCCCCATCCTAGAACCAGAG | AGG | 27,8841387 | 73,994295 |
| 415 | 154274993 | -1 | AGACTTACCATTGTCCTCTC | TGG | 39,329239 | 44,5272658 |
| 416 | 154274997 | 1 | CCTAGAACCAGAGAGGACAA | TGG | 34,5134102 | 69,7146312 |
| 417 | 154275006 | 1 | AGAGAGGACAATGGTAAGTC | TGG | 39,8124086 | 51,4865492 |
| 418 | 154275009 | 1 | GAGGACAATGGTAAGTCTGG | AGG | 39,7778548 | 62,6787204 |
| 419 | 154275013 | 1 | ACAATGGTAAGTCTGGAGGA | AGG | 34,7929273 | 60,7439937 |
| 420 | 154275014 | 1 | CAATGGTAAGTCTGGAGGAA | GGG | 33,9071193 | 49,763306 |
| 421 | 154275015 | 1 | AATGGTAAGTCTGGAGGAAG | GGG | 30,4586522 | 50,1112907 |
| 422 | 154275034 | -1 | TCAGAAGAATAACAAAAGGC | TGG | 30,7277575 | 55,8366745 |
| 423 | 154275038 | -1 | AACTTCAGAAGAATAACAAA | AGG | 27,707663 | 49,6573695 |
| 424 | 154275072 | -1 | ACAGGAAGAAACTTCAGGGA | GGG | 27,1894341 | 66,2958724 |
| 425 | 154275073 | -1 | TACAGGAAGAAACTTCAGGG | AGG | 32,3692237 | 68,6631191 |
| 426 | 154275076 | -1 | GTGTACAGGAAGAAACTTCA | GGG | 35,6405331 | 59,6221348 |
| 427 | 154275077 | -1 | AGTGTACAGGAAGAAACTTC | AGG | 33,5802811 | 42,2497987 |
| 428 | 154275090 | -1 | AAAAAAGGAGACAAGTGTAC | AGG | 36,243166 | 55,7658482 |
| 429 | 154275105 | -1 | GAAAGAGTCCAAAGGAAAAA | AGG | 23,6136104 | 35,2921766 |
| 430 | 154275108 | 1 | ACTTGTCTCCTTTTTTCCTT | TGG | 21,2700233 | 25,918552 |
| 431 | 154275113 | -1 | GCAGGAGAGAAAGAGTCCAA | AGG | 33,6451505 | 61,8741325 |
| 432 | 154275131 | -1 | GAGCAGAGAGATGAAGAAGC | AGG | 27,2645908 | 52,1833585 |
| 433 | 154275156 | -1 | GAAACCTGGGAATCAAGATC | TGG | 38,1402956 | 46,3655718 |
| 434 | 154275163 | 1 | TCTTCCAGATCTTGATTCCC | AGG | 38,6067711 | 45,6977978 |
| 435 | 154275169 | -1 | AAGACCCTCCTGGGAAACCT | GGG | 33,1936838 | 63,6687824 |
| 436 | 154275170 | -1 | CAAGACCCTCCTGGGAAACC | TGG | 23,3014309 | 40,1286987 |
| 437 | 154275172 | 1 | TCTTGATTCCCAGGTTTCCC | AGG | 29,4897615 | 48,9333777 |
| 438 | 154275175 | 1 | TGATTCCCAGGTTTCCCAGG | AGG | 31,6791996 | 59,1993118 |
| 439 | 154275176 | 1 | GATTCCCAGGTTTCCCAGGA | GGG | 25,3096829 | 53,4952435 |
| 440 | 154275178 | -1 | AACCGGGCCAAGACCCTCCT | GGG | 36,9009862 | 56,4228071 |
| 441 | 154275179 | -1 | GAACCGGGCCAAGACCCTCC | TGG | 38,8623551 | 49,4210907 |
| 442 | 154275182 | 1 | CAGGTTTCCCAGGAGGGTCT | TGG | 29,8069332 | 41,0239394 |
| 443 | 154275187 | 1 | TTCCCAGGAGGGTCTTGGCC | CGG | 29,7819344 | 44,8453822 |
| 444 | 154275194 | -1 | TGGGCTGGGGCTGTAGAACC | GGG | 28,0703824 | 50,0593626 |
| 445 | 154275195 | -1 | TTGGGCTGGGGCTGTAGAAC | CGG | 27,8122597 | 35,952732 |
| 446 | 154275207 | -1 | TTGAAATAGGATTTGGGCTG | GGG | 35,9760513 | 44,3703032 |
| 447 | 154275208 | -1 | CTTGAAATAGGATTTGGGCT | GGG | 37,7355775 | 43,92758 |
| 448 | 154275209 | -1 | TCTTGAAATAGGATTTGGGC | TGG | 40,9877748 | 51,8389594 |
| 449 | 154275213 | -1 | ATGCTCTTGAAATAGGATTT | GGG | 35,2406437 | 33,3983015 |
| 450 | 154275214 | -1 | GATGCTCTTGAAATAGGATT | TGG | 36,3894949 | 28,3548159 |
| 451 | 154275220 | -1 | CACAGAGATGCTCTTGAAAT | AGG | 35,0992989 | 33,9658185 |
| 452 | 154275244 | -1 | CCCATCTGGTTTAGTGATCT | TGG | 43,1898122 | 31,0299156 |
| 453 | 154275254 | 1 | ACCAAGATCACTAAACCAGA | TGG | 39,4792861 | 53,8961826 |
| 454 | 154275255 | 1 | CCAAGATCACTAAACCAGAT | GGG | 40,9539154 | 53,6827765 |
| 455 | 154275256 | 1 | CAAGATCACTAAACCAGATG | GGG | 40,4938448 | 63,3135289 |
| 456 | 154275258 | -1 | TTCTTTCAACTCACCCCATC | TGG | 39,6115031 | 28,5416128 |
| 457 | 154275274 | 1 | TGGGGTGAGTTGAAAGAAAG | AGG | 30,1722522 | 59,7114084 |
| 458 | 154275280 | 1 | GAGTTGAAAGAAAGAGGTAA | AGG | 28,7458632 | 50,9731655 |
| 459 | 154275289 | 1 | GAAAGAGGTAAAGGAAAGTA | TGG | 28,1324074 | 45,4679617 |
| 460 | 154275294 | 1 | AGGTAAAGGAAAGTATGGCC | AGG | 38,1949594 | 51,016487 |
| 461 | 154275295 | 1 | GGTAAAGGAAAGTATGGCCA | GGG | 37,2806318 | 59,7917161 |
| 462 | 154275301 | -1 | GTTTCAGGGCATTCGTTCCC | TGG | 45,9816352 | 41,2627136 |
| 463 | 154275315 | 1 | GGGAACGAATGCCCTGAAAC | AGG | 43,8674793 | 41,1321618 |
| 464 | 154275315 | -1 | TTACACAGATCCCTGTTTCA | GGG | 35,9611689 | 38,7591239 |
| 465 | 154275316 | 1 | GGAACGAATGCCCTGAAACA | GGG | 41,4276643 | 54,2374774 |
| 466 | 154275316 | -1 | TTTACACAGATCCCTGTTTC | AGG | 35,7426382 | 17,3843559 |
| 467 | 154275356 | -1 | CATATTCCGAATGTTACTAA | AGG | 40,6058012 | 52,3526226 |
| 468 | 154275361 | 1 | GCATAACCTTTAGTAACATT | CGG | 36,1655001 | 46,8531298 |
| 469 | 154275368 | 1 | CTTTAGTAACATTCGGAATA | TGG | 45,8426179 | 33,4447172 |
| 470 | 154275371 | 1 | TAGTAACATTCGGAATATGG | TGG | 42,956761 | 54,8498648 |
| 471 | 154275372 | 1 | AGTAACATTCGGAATATGGT | GGG | 42,0460307 | 56,1385155 |
| 472 | 154275373 | 1 | GTAACATTCGGAATATGGTG | GGG | 44,3272284 | 63,0796496 |
| 473 | 154275394 | 1 | GGACTTCTCTTTGTAGATAG | TGG | 40,3843351 | 51,5958762 |
| 474 | 154275397 | 1 | CTTCTCTTTGTAGATAGTGG | AGG | 39,0878226 | 58,3477774 |
| 475 | 154275405 | 1 | TGTAGATAGTGGAGGAGCGC | CGG | 43,4653933 | 52,3839909 |
| 476 | 154275412 | 1 | AGTGGAGGAGCGCCGGACTG | TGG | 42,9098582 | 58,7596601 |
| 477 | 154275413 | -1 | CCTCACTGTCCACCACAGTC | CGG | 27,1288152 | 44,8106272 |
| 478 | 154275415 | 1 | GGAGGAGCGCCGGACTGTGG | TGG | 34,1318485 | 54,8730489 |
| 479 | 154275424 | 1 | CCGGACTGTGGTGGACAGTG | AGG | 26,5639033 | 53,465173 |
| 480 | 154275425 | 1 | CGGACTGTGGTGGACAGTGA | GGG | 26,1700772 | 50,2290282 |
| 481 | 154275429 | 1 | CTGTGGTGGACAGTGAGGGC | CGG | 23,7168155 | 53,6230449 |
| 482 | 154275437 | -1 | GGGTTACTGTAGTCTCTGTC | CGG | 42,6374812 | 47,7595483 |
| 483 | 154275457 | -1 | ACTGCTATCTGCTTCGTGTC | GGG | 38,081181 | 44,8426181 |
| 484 | 154275458 | -1 | GACTGCTATCTGCTTCGTGT | CGG | 31,0666949 | 44,3484667 |
| 485 | 154275474 | 1 | ACGAAGCAGATAGCAGTCCT | AGG | 28,9513867 | 51,9932372 |
| 486 | 154275475 | 1 | CGAAGCAGATAGCAGTCCTA | GGG | 43,7428353 | 42,6853866 |
| 487 | 154275476 | 1 | GAAGCAGATAGCAGTCCTAG | GGG | 41,1915783 | 64,3435967 |
| 488 | 154275479 | 1 | GCAGATAGCAGTCCTAGGGG | TGG | 42,0805222 | 62,99955 |
| 489 | 154275480 | -1 | TCTTTTAACTTACCACCCCT | AGG | 40,9978458 | 55,9292043 |
| 490 | 154275497 | 1 | GGTGGTAAGTTAAAAGACAA | AGG | 33,2011847 | 63,6194069 |
| 491 | 154275498 | 1 | GTGGTAAGTTAAAAGACAAA | GGG | 28,6894704 | 44,1887359 |
| 492 | 154275499 | 1 | TGGTAAGTTAAAAGACAAAG | GGG | 27,9906819 | 69,3559264 |
| 493 | 154275519 | 1 | GGGTTCATCTCAAGATTCCT | TGG | 42,0998457 | 40,6811639 |
| 494 | 154275520 | 1 | GGTTCATCTCAAGATTCCTT | GGG | 40,0929998 | 42,7379736 |
| 495 | 154275521 | 1 | GTTCATCTCAAGATTCCTTG | GGG | 37,3383475 | 67,1681719 |
| 496 | 154275525 | 1 | ATCTCAAGATTCCTTGGGGA | AGG | 36,2349598 | 49,6588563 |
| 497 | 154275525 | -1 | AGTAAGATTTCCCTTCCCCA | AGG | 32,8635903 | 64,3998232 |
| 498 | 154275526 | 1 | TCTCAAGATTCCTTGGGGAA | GGG | 34,7697447 | 57,2176967 |
| 499 | 154275554 | -1 | TAGGCAGAAGCAAAGGACAA | GGG | 29,3013711 | 68,827079 |
| 500 | 154275555 | -1 | CTAGGCAGAAGCAAAGGACA | AGG | 30,2754731 | 55,3590827 |
| 501 | 154275561 | -1 | AAGAGACTAGGCAGAAGCAA | AGG | 32,4955176 | 63,1644501 |
| 502 | 154275573 | -1 | TAGGCTAAATGAAAGAGACT | AGG | 31,3904328 | 58,8315515 |
| 503 | 154275592 | -1 | AGAAAGTCATACACGTAAAT | AGG | 43,935994 | 25,7713974 |
| 504 | 154275616 | -1 | TCTTGGTGATTCTGGATCTA | AGG | 40,6811479 | 45,2001903 |
| 505 | 154275624 | -1 | GCTGGAGGTCTTGGTGATTC | TGG | 29,3106053 | 23,0411682 |
| 506 | 154275633 | -1 | TCATCCAGGGCTGGAGGTCT | TGG | 27,4643151 | 32,9071435 |
| 507 | 154275639 | -1 | AAGGCATCATCCAGGGCTGG | AGG | 30,1838865 | 46,1180304 |
| 508 | 154275640 | 1 | ATCACCAAGACCTCCAGCCC | TGG | 27,4904196 | 44,0524016 |
| 509 | 154275642 | -1 | GAAAAGGCATCATCCAGGGC | TGG | 34,5378331 | 42,0387869 |
| 510 | 154275646 | -1 | GATGGAAAAGGCATCATCCA | GGG | 29,0185866 | 48,0016899 |
| 511 | 154275647 | -1 | GGATGGAAAAGGCATCATCC | AGG | 39,9624844 | 51,9632722 |
| 512 | 154275658 | -1 | GAATAAGTCCAGGATGGAAA | AGG | 26,5773263 | 44,2085034 |
| 513 | 154275661 | 1 | GGATGATGCCTTTTCCATCC | TGG | 31,6125569 | 34,3020588 |
| 514 | 154275664 | -1 | TCCCAGGAATAAGTCCAGGA | TGG | 26,5030926 | 45,9055071 |
| 515 | 154275668 | -1 | AACGTCCCAGGAATAAGTCC | AGG | 42,5433872 | 46,1329916 |
| 516 | 154275673 | 1 | TTCCATCCTGGACTTATTCC | TGG | 28,846623 | 6,58631807 |
| 517 | 154275674 | 1 | TCCATCCTGGACTTATTCCT | GGG | 27,5373885 | 20,4357559 |
| 518 | 154275680 | -1 | GGGACCGGAACCAACGTCCC | AGG | 47,3689036 | 31,3883626 |
| 519 | 154275681 | 1 | TGGACTTATTCCTGGGACGT | TGG | 45,5158697 | 30,48055 |
| 520 | 154275687 | 1 | TATTCCTGGGACGTTGGTTC | CGG | 46,1147768 | 25,3099535 |
| 521 | 154275693 | 1 | TGGGACGTTGGTTCCGGTCC | CGG | 47,6693649 | 26,3815168 |
| 522 | 154275695 | -1 | GTTAACAAGGCTACCGGGAC | CGG | 31,6065467 | 27,9551005 |
| 523 | 154275700 | -1 | TGAGGGTTAACAAGGCTACC | GGG | 30,8988454 | 36,4484683 |
| 524 | 154275701 | -1 | CTGAGGGTTAACAAGGCTAC | CGG | 30,3313152 | 26,9148939 |
| 525 | 154275708 | -1 | AAGGCCTCTGAGGGTTAACA | AGG | 28,3695765 | 52,57064 |
| 526 | 154275715 | 1 | GTAGCCTTGTTAACCCTCAG | AGG | 30,9060195 | 67,9925555 |
| 527 | 154275717 | -1 | AAGGACTTGAAGGCCTCTGA | GGG | 26,2821992 | 62,3738685 |
| 528 | 154275718 | -1 | AAAGGACTTGAAGGCCTCTG | AGG | 26,0136497 | 63,0739114 |
| 529 | 154275727 | -1 | GAGAGGTGGAAAGGACTTGA | AGG | 25,3051586 | 48,8437966 |
| 530 | 154275736 | -1 | GCAATGGGTGAGAGGTGGAA | AGG | 23,4627517 | 60,1064709 |
| 531 | 154275741 | -1 | GGTGGGCAATGGGTGAGAGG | TGG | 23,597536 | 63,3788668 |
| 532 | 154275744 | -1 | AATGGTGGGCAATGGGTGAG | AGG | 24,4806215 | 55,5686301 |
| 533 | 154275751 | -1 | GCTTATTAATGGTGGGCAAT | GGG | 30,4347738 | 47,8496264 |
| 534 | 154275752 | -1 | AGCTTATTAATGGTGGGCAA | TGG | 33,6558187 | 46,9620535 |
| 535 | 154275758 | -1 | AAGCTAAGCTTATTAATGGT | GGG | 36,4950905 | 62,6929627 |
| 536 | 154275759 | -1 | GAAGCTAAGCTTATTAATGG | TGG | 38,2596968 | 62,6898526 |
| 537 | 154275762 | -1 | AGAGAAGCTAAGCTTATTAA | TGG | 36,3489898 | 30,6421531 |
| 538 | 154275785 | 1 | TTAGCTTCTCTTGCCACCTC | AGG | 37,2689649 | 29,464469 |
| 539 | 154275786 | 1 | TAGCTTCTCTTGCCACCTCA | GGG | 37,4454838 | 46,7040828 |
| 540 | 154275787 | 1 | AGCTTCTCTTGCCACCTCAG | GGG | 36,2165788 | 52,8920359 |
| 541 | 154275787 | -1 | ACATATCCAAGCCCCTGAGG | TGG | 38,1574324 | 63,2976638 |
| 542 | 154275790 | -1 | TCCACATATCCAAGCCCCTG | AGG | 36,5762386 | 68,7050622 |
| 543 | 154275792 | 1 | CTCTTGCCACCTCAGGGGCT | TGG | 36,3462349 | 34,6540359 |
| 544 | 154275800 | 1 | ACCTCAGGGGCTTGGATATG | TGG | 39,8331819 | 47,0930402 |
| 545 | 154275813 | 1 | GGATATGTGGAATAGTGAAC | TGG | 40,8228787 | 45,3626808 |
| 546 | 154275814 | 1 | GATATGTGGAATAGTGAACT | GGG | 40,9936694 | 60,0229738 |
| 547 | 154275815 | 1 | ATATGTGGAATAGTGAACTG | GGG | 38,3124885 | 79,7389664 |
| 548 | 154275827 | -1 | GGGTGAGTGACAAACTGACA | TGG | 39,4053304 | 71,1843369 |
| 549 | 154275847 | -1 | AAGGTTTTATTGGTCAGTTT | GGG | 26,394879 | 38,4452392 |
| 550 | 154275848 | -1 | AAAGGTTTTATTGGTCAGTT | TGG | 27,1317023 | 34,7921285 |
| 551 | 154275857 | -1 | AGCATAAATAAAGGTTTTAT | TGG | 20,6078658 | - |
| 552 | 154273432 | | GCTGAGGGCTATGGAACCTT (sgHAX1.PS-SNP) | GGG | | |
| 553 | 154273432 | | GCTGAGGACTATGGAACCTT (HAX1 Ex 2) | GGG | | |
| 554 | Chr19:55115581* | | CTCCCTCCCAGGATCCTCTC (AAVS1) | TGG | | |

**Table 2: Primers**

| SEQ ID NO: | PCR Primers | Sequence |
|---|---|---|
| 555 | AAVS1 primer fwd | CACCTTATATTCCCAGGGCCG |
| 556 | AAVS1 primer rev | CCTAGGACGCACCATTCTCAC |
| 557 | HAX1 AAV template primer fwd | TCCAGCCTGGGAAACATGAGAG |
| 558 | HAX1 AAV template primer rev | ACTACTCCCGAGCTCCAAAACC |
| | | |
| | Sanger sequencing primer | |
| 559 | AAVS1 primer fwd | CACCTTATATTCCCAGGGCCG |
| 560 | HAX1 primer fwd | CCATGAGTTGATTTAATGGCTTA |
| | | |
| | Primers for HDR template generation | |
| 561 | Phos-HAX1-AAVHDR-F | /5Phos/ACATCGCCCAGGTCAGTCAAAAAG |
| 562 | Phos-HAX1-AAVHDR-R | /5Phos/AAACACTAAATGGAGGGCTTAG |
| | | |
| 563 | SDM.HAX1.G.CT.FWD | GAGGACTATGGAACCTAGGATTGCCGCGTCCCC ATGAGCCCCCTT |
| 564 | SDM.HAX1.G.CT.REV | AAGGGGGCTCATGGGGACGCGGCAATCCTAGGT TCCATAGTCCTC |

**Table 3: HAX1 Correction template delivered by AAV**

| |
|---|
| ACATCGCCCAGGTCAGTCAAAAAGAACGCCCGAGTAAGGAGCGAGGTGGCTTCTGCTGTGAGGCTGGC CAGCCCCGCGGGTGGGGTGGTTGAGGGACGCCCTCAAGCCGGTCCATTTGTGCGGCTGGGCTGCCCT CTGGCGGCTTTATCCGGGTGCCATCCAGAGTCAAACCCTCGAAATCGCTTTTCGTACTGAGTTGGTCTGC AGAAAGAAGAACAGATTTAGGAGGAAGGCGGTCATAGGAAACTGAAAGTGCGTCACGCCCACTCCCGTC ACGGAAACTGGTCTCTGAAAGGTGGGGTAAAGATAAAGCCTTTCAAATTTGAGGAAGACCTTCGGTCCCG CCTCCATTTCACGTCCGGCTTACCGTCGTTTACGACAGTGTCAGGATCGCGGGCTTGCTTTCCGGTAGC GTGGGCTGACGCCTCGCTCAATTTCTCACAGGGCTGCGCAGGTTTCCCCCGTCTGCGAATGGACCACTG GAGGGGTTCAAAGGTTCGCGTCCCAGTACGGGAATGAGCCTCTTTGATCTCTTCCGGGGCTTTTTCGGC TTTCCTGGACCTCGGAGGTGAGAGTAGGTCCGGCTCGGACAAGGGTGGGGGTCGTCTGAGGGGAGCTT GACCCCTACGTCTTATTTTTGGAAAAACATCCTCTGTCCCACTCCTTCAACTCCTGCAGAGAGGACAGAA GTCGCAACATTGAACACTCACCCCGCCACAGTAACCAAGGCGGTCATTAAGAGGAGAAACAGCAAACTA AGCCTTTCTCCAACCTGGGGTGATGCAACAGGGACCCGGGCGGGGAAGACCGTGAGGGTCTGGGGAAT AAGACAGTGAGCAAGTGAGCAGGACCTTGGGAGAGAGAGGAGGGACTGGGGCACACTGAAGAAAAACT GGGGGAAGGTGTATGAAGGGAGCTGCGAGCTGAGGTCTGACTTT- GATTaaaaaaaaaaaaaaaaaaaaaGAACTGTCAGCCATTGTATTAATGTTTTGAT- GTGGCAGCCAGTCCTCCGACCCTCTCCCTAGCTTCCCAGACCCCTTGCTCTTGTCCCACTTTGCCACCCA TGAGTTGATTTAATGGCTTAAATAGTGCTGAAATATTGGTGGCCAATCTGCCTCCACTCTCAGCCACAGAG |
| ATCCCTTTTTTGGAGGGATGACTCGAGATGAAGATGATGATGAGGAAGAAGAAGAAGGGGGCTCAT GGGGACGCGGCAATCCTAGGTTCCATAGTCCTCAGCACCCCCCTGAGGAATTTGGCTTCGGCTTCAGCT TCAGCCCAGGAGGAGGGATACGTTTCCACGATAACTTCGGCTTTGATGACCTAGTACGAGATTTCAATAG CATCTTCAGCGATATGGGGGCCTGGACCTTGCCTTCCCATCCTCCTGGTGTGTGGCTTTCCCTAAGGGG CAACCTGTGGTTTCTGGTGGGTTGGTGGGTGAAATAAAGAGCCTGCAGGGAGTAGCTGGGGGATGGGA AGTGTGAGAAGACTGATGATTTCAGAGAGATTAATAGAGCCCAAGTCCTTTCCCATCCCAGCAAACACCT GCCACCTTTCTGCAGAACTTCCAGGTCCTGAGTCAGAGACACCTGGTGAGAGACTACGGGAGGGACAGA CACTTCGGGACTCAATGCTTAAGTATCCAGATAGTCACCAGCCCAGGATCTTTGGGGGGGTCTTGGAGA GTGATGCAAGAAGTGAATCCCCCCAACCAGCACCAGACTGGGGCTCCCAGAGGCCATTTCATAGGGTGA GTATCCCATCTGGTCCTGAAGTGAGAGCTTGTGAGAGACCA- CTAATAAAGTGCAAAGACTggctaggtgcggtggctcacgcctgtaatcccagcactttgg- gaggccgaggtgggcagatcaccagaggtcaggagttggagaccagcctggtcaacatggt- gaaaccccgtgtctactaaaaatacaaaaaattagccgggcatggtggcaggtgcctgtaat- gccagctactcaggaggctgaggcaggagaattggttgaacccaggaggtggagACTCCATCT- CAAAAAttgtttgagacccagccttgctctgttgcccaggctgtagtgccgtggcacaatct- cagctcactgcaacctccaattcccaggttcaagtgattctcctgcctcagcctcctgag- tagctgggattacagtcgcctgccaaacgcccagctaatttttgtgtttttagtagagat- ggcgtttcaccgtgttggccaggctgcttttgagctcctgacctcaggtgatccacccac- ctcggcctcccaaagtgctggaattacaagtgtgagccatcatgcctggccAAAGATT- GCAATTCTTGTTTGAATCTGATAGCCTTGGGTTGGAATCCTAAGCCCTCCATTTAGTGTTT (SEQ ID NO: 565) |

### An ex vivo selection-free gene therapy approach to correct HAX1 mutation p. W44X using CRISPR/Cas9 gene-editing

The inventors established an ex vivo gene-therapy approach to correct the mutation p.W44X (c.131insA) in the *HAX1* gene (Figure 1A). To this end, the inventors used the Cas9 nuclease with a chemically modified sgRNA (sgHAX1.PS-SNP; SEQ ID NO: 552) to introduce a double-strand break close to the *HAX1* mutated site in HSPCs. The custom guide RNA induces a cut inside the codon at the amino acid position p.R50 of the wildtype protein sequence. Subsequently, rAAV6 is used to deliver the designed homologous recombination-based repair template (SEQ ID NO: 565). The HDR template includes five silent mutations between the *HAX1* mutation and the cut site to increase gene-editing efficiency by preventing recurrent cutting of correctly edited alleles and premature HDR termination (Figure 1B, C). The developed approach is selection free, as the guide RNA targets a position downstream of the mutation to delete the adenosine responsible for the stop codon causing *HAX1-CN* (Figure 1B, C). Unwanted indels remain silent, as the stop codon prevents *de novo* protein synthesis that could arise due to indels. This approach would result in an *in situ* correction of the *HAX1* gene preserving timing and splicing of gene expression.

### CRISPR/Cas9-based HAX1 knockout in HSPCs of healthy individuals: a novel in vitro model of HAX1-CN

Since *HAX1-CN* is a rare disease and primary cells are difficult to obtain, the development of suitable models to test drugs or develop gene therapy approaches is a key step for clinical translation. The inventors reasoned that Cas9-mediated editing of *HAX1* in exon 2 would induce a HAX1-CN phenotype in healthy HSPCs, and the combination of Cas9 with the repair template would rescue the phenotype in these edited HSPCs. Therefore, in order to test the inventors' ex *vivo* gene therapy approach, the inventors first generated *HAX1* knockout (KO) HSPCs derived from healthy donors. To this end, the inventors designed sgRNAs targeting exon 2 of *HAX1* (HAX1 Ex 2; SEQ ID NO: 553) using CRISPOR (Concordet J-P, Haeussler M. (2018), CRISPOR: intuitive guide selection for CRISPR/Cas9 genome editing experiments and screens. Nucleic Acids Research 46(W1): W242-W5). Among all potential guides within a distance of 30 base pairs from the mutation site, the inventors selected sgRNAs with the lowest off-target activity using CRISPRitz (Cancellieri et al. (2019), CRISPRitz: rapid, high-throughput and variant-aware in silico off-target site identification for CRISPR genome editing. Bioinformatics 2019; 36(7): 2001-8.) (Figure 2A). All *HAX1-CN* patients screened in the inventors' lab in the past and all patients used in this study had the single nucleotide polymorphism (SNP) rs13796 (dbSNP build 150). Therefore, the inventors *inter alia* designed a sgRNA with the corresponding base at that position (sgHAX1.PS-SNP; SEQ ID NO: 552) (Figure 1B, C & Figure 2B; Table 1).

Once the inventors established a CRISPR/Cas9-based *HAX1* KO in HSPCs, the inventors further tested the *HAX1* gene-editing approach in HSPCs from healthy donors (Figure 1D). To control the double-strand break independent homologous recombination of the repair template, the inventors targeted the *AAVS1* safe harbor locus (HD *AAVS1* control), transducing the cells with rAAV6 containing the correction template (SEQ ID NO: 565) (Figure 3A). HSPCs electroporated with sgRNA targeting *HAX1* (sgHAX1.PS-SNP) were transduced with either a control rAAV6 containing the *HAX1* HDR template (HD *HAX1* corrected) or no HDR template (HD *HAX1* KO). After 72 hours post-transduction, the inventors determined gene-editing efficiency using Sanger sequencing traces and the ICE webtool (Analysis SP. ICE Analysis. 2019 (accessed 29.09.2020)). The inventors achieved 82.0 % (± 1,41 %) total editing (TE) in HD *AAVS1* control cells, 86.0 % (± 5.66 %) in HD *HAX1* KO cells, and 88.5% (± 0.71 %) in HD *HAX1* corrected cells (Figure 1E). The knockout (KO) frequency was 80.0 % (± 4.24 %) in HD *HAX1* KO cells, and the knock-in (KI) frequency in HD *HAX1* corrected cells was 33.5 % (± 6.36 %) (Figure 1E).

Next, the inventors evaluated the efficiency of their approach by differentiating gene-edited CD34⁺ HSPCs ex *vivo.* The TE rate remained constant over the 14 days of ex *vivo* differentiation at 80.3 % (± 0.99 %) in HD *AAVS1* control cells. In HD *HAX1* KO cells, the percentage of TE and KO declined to 71.5 % (± 0.7 %, *p* = 0.0693) and 55.0 % (± 5.66 %, *p* = 0.037), respectively. In HD *HAX1* corrected group, TE declined significantly to 76.5 % (± 0.71 %, *p* = 0.0035), but the KI efficiency declined slightly to 29.5 % (± 4.95 %, *p* = 0.556), representing a clonal advantage of *HAX1* corrected over the *HAX1* KO cells (Figure 1E).

When the inventors determined the distribution of differentiated cells by flow cytometry, they observed an increase of immature myeloid cells consisting of myeloid blasts and promyelocytes in HD *HAX1* KO cells. Simultaneously, the number of metamyelocytes and mature neutrophils was reduced when compared to HD *AAVS1* control group (Figure 4A, B). The changes observed in HD *HAX1* KO cells returned to wild-type levels in *HAX1* corrected cells (Figure 4A, B). Differentiation was also assessed by morphological analysis of Wright-Giemsa stained cytospins. This analysis confirmed that *HAX1* KO induced a differentiation blockade increasing the number of immature and intermediate matured granulocytes, whereas HD *HAX1* corrected group showed restored granulocytic differentiation to near wild-type levels (Figure 4C-E). These findings indicate that *HAX1* knockout in exon two at amino acid position p.R50X can be used to model *HAX1-CN* mutation p.W44X. Additionally these results show a correction of the mutation and therefore a rescue of the CN-phenotype, suggesting the potential of this approach as a gene therapy for *HAX1-CN* patients.

### Efficient correction of HAX1 mutations in HSPCs of HAX1-CN patients

In order to confirm the inventors' observations from those studies in healthy HSPCs, they further tested the *HAX1* gene-editing approach in primary HSPCs from 5 CN patients harboring the *HAX1* mutation p.W44X (Figure 5A, B). In the control group edited with the sgRNA targeting the *AAVS1* locus and transduced with the *HAX1-*HDR template (CN AAVS1 control), the editing in HSPCs amounted to 76.74 % (± 17.07 %). In the group edited with the sgRNA HAX1.PS-SNP (SEQ ID NO: 552) and transduced with *HAX1*-HDR template (SEQ ID NO: 565) (CN *HAX1* corrected), the inventors observed TE of 84.4 % (± 4.2 %) and KI of 65.8 % (± 7.12 %) (Figure 5A). After ex *vivo* differentiation of gene-edited cells to neutrophils, the inventors observed constant TE with 73.3 % (± 15.47 %) in the CN *AAVS1* control group and 83.4 % (± 7.37 %) in the CN *HAX1* corrected group. The frequency of corrected alleles, on the other hand, increased absolutely by 10 % to 75.8 % (± 7.918 %). In order to confirm that *HAX1* gene-editing restored HAX1 protein expression, the inventors performed Western Blot analysis of differentiated edited cells detecting HAX1 protein in CN *HAX1* corrected cells, whereas the CN *AAVS1* cells lacked expression of HAX1 protein (Figure 5C).

### Improved in vitro granulocytic differentiation in gene-edited HSPCs of HAX1-CN patients

To assess if the editing efficiency was sufficient to rescue the CN phenotype, the inventors investigated the ex *vivo* neutrophil differentiation of gene-edited cells. The inventors observed that corrected HSPCs generated on average two times more cells than control edited cells. The number of CN *AAVS1* control cells increased on an average of 2.78-fold (± 1.04) compared to a significantly higher 5.6-fold increase (± 2.82, *p =* 0.0424) of CN *HAX1* corrected HSPCs, as compared to day 1 of culture (Figure 6A). The morphological analysis of Wright-Giemsa stained cytospins of differentiated cells revealed a significantly increased proportion of mature granulocytes in CN *HAX1* corrected cells (p = 0.005), which was in line with decreased amounts of immature myeloid cells and apoptotic cells, as compared to CN *AAVS1* control groups (Figure 6B, C). Performing flow cytometry analysis of granulocytic differentiation, the inventors observed a significant 2.5-fold increase of mature neutrophils (p = 0,008) and a significant (p = 0.012) 1.5-fold reduction of promyelocytes (Figure 6D). These data argue the successful correction of the maturation arrest of granulopoiesis *in vitro* by CRISPR/Cas9 mediated correction of *HAX1* mutation in CN HSPCs.

### Correction of HAX1 mutation protects CN HSPCs from intrinsic stress

The inventors have recently described an increased sensitivity of *HAX1-*CN HSPCs to oxidative stress. To investigate whether the correction of *HAX1* mutation and the reinstated expression of HAX1 protein protect HSPCs from oxidative stress, the inventors performed live cell imaging of H₂O₂-induced caspase3/7 activation. The inventors detected a substantial reduction of H₂O₂-triggered apoptosis in ex *vivo* differentiated cells of CN *HAX1* corrected HSPCs when compared to CN *AAVS1* control (Figure 7A).

Furthermore, CN *HAX1* corrected differentiated cells were investigated for the functional hallmarks of granulocytes. The inventors observed that CN *HAX1* corrected granulocytes of two *HAX1-CN* patients showed increased phagocytosis of E. *coli* bioparticles when compared to CN *AAVS1* control (Figure 7B & 8A). Additionally, the differentiated cells generated from CN *HAX1* corrected cells showed an increased capability to produce neutrophil extra cellular traps (NETs) upon stimulation with phorbol myrstate acetate (PMA), compared to differentiated CN *AAVS* control cells (Figure 7C & 9A, B). Chemotaxis was also markedly increased in CN *HAX1* corrected cells (Figure 10A). Using a luminescence assay detecting luciferin's activation in dependency to reactive oxygen species (ROS), we measured ROS production upon stimulation of cells with N-formylmethionine-leucil-phenylalanine (fMLP). The amount of ROS produced in control cells and upon fMLP stimulation was higher in CN *HAX1* corrected compared to CN *AAVS1* control samples (Figure 8B). However, the inventors observed no difference between CN *AAVS1* control and CN *HAX1* corrected cells in ROS production fold-change from control cells to fMLP activated cells (Figure 7D). In conclusion, the gene correction of *HAX1* exon 2 produced functional mature neutrophils with increased resistance to H₂O₂-triggered apoptosis as well as improved phagocytic capacity, production of NETs and chemotaxis compared to CN *AAVS1* control edited cells.

### 4. Discussion

For the first time, the inventors described a CRISPR/Cas9-based correction of autosomal recessive *HAX1* mutations in primary HSPCs of CN patients. The inventors achieved high correction efficiency that resulted in the desired restoration of HAX1 protein expression and functions since the inventors observed markedly improved granulocytic differentiation of corrected cells ex *vivo.* It is sufficient for autosomal recessive disorders to correct one mutated allele. For the loss-of-function mutations (*HAX1* mutations) resulting in the absent protein expression, the probability of introducing pathological truncated proteins as byproducts of NHEJ is extremely low. With the inventors' approach, unwanted indels will not be translated since they are located downstream of the stop codon. The inventors believe that their gene therapy approach for *HAX1-CN* using HDR-based mutation correction is highly efficient, safe, and therefore perfectly suitable for clinical applications as ex *vivo* gene therapy.

CRISPR/Cas9 might introduce novel missense or in-frame mutations or large deletions that were not initially found in *HAX1-CN.* The role of these novel *HAX1* mutations in granulopoiesis and leukemogenesis should be evaluated. Since the inventors did not detect such mutations in gene-edited cells, simultaneously observing improved granulocytic differentiation and resistance to H₂O₂-induced apoptosis, the inventors assumed that these mutations appear at a neglectable frequency, not affecting these processes. Long-term effects of novel missense or frameshift *HAX1* mutations introduced by CRISPR/Cas9 editing should be evaluated *ex vivo* using long-term HSC (LT-HSC) culture and *in vivo* using engraftment of HSPCs in immunodeficient NSG mice.

Another more general gene-editing approach for the correction of inherited gene mutations is based on the introduction of full-length wild-type cDNA, disrupting the transcription initiation site of the mutated cDNA. This approach is not suitable for the restoration of mutated *HAX1* expression due to the specific expression of at least eight different *HAX1* isoforms in different mature blood cell subtypes with yet to be determined functions. Some of these eight HAX1 isoforms are already known to contribute to certain cancers. Introduction of the wild-type full-length *HAX1* cDNA instead of restoring the reading frame would affect the expression of these *HAX1* variants produced by alternative splicing. The exclusive expression of the full-length isoform might impair specific cell functions dependent on particular *HAX1* isoforms or even support or enable malignant transformation of targeted cells. The two iPSC studies that have tested the ectopic expression of HAX1 by lentiviral vectors with inconsistent results support the importance of HAX1 isoforms.

The inventors used AAV-based transduction to deliver intracellularly an HDR template of *HAX1.* This method is already used for gene-editing of HSPCs followed by autologous transplantation of gene-corrected cells in clinical trials for sickle cell anemia and has been extensively studied. Although relatively safe and currently the most efficient way to deliver a HDR-template without triggering the innate immune system of HSPCs, AAV-based delivery might induce unwanted cellular and intracellular responses to AAV particles. As an alternative approach, other nucleic acid delivery methods, such as modified nucleic acid, nanoparticle- or extracellular vesicles (EV) - based cellular transport, might be used. Additionally, the aspect of selecting p53 defective HSCs through double-strand break editing approaches might be essential to consider in patients that have already increased risk to develop hematological malignancies. Here, recent advances that suppress DNA damage response through p53 and innate immune response in HSC during genome editing and increase clonality can be applied to the strategy of the inventors. Alternatively, gene-editing strategies that do not rely on double-strand breaks and can delete the pathogenic adenosine, like editing editing, or another approach, might be a good option once they are refined and more broadly accessible.

To provide curative therapy, the inventors need to gene-edit multipotent long-term HSCs (LT-HSCs). These cells are essential for the multilineage long-term reconstitution of hematopoiesis after gene therapy. The main hurdle is the low proliferation rate of these cells, an essential pre-requisite for HDR after CRISPR/Cas9 gene-editing. There are several suggestions for targeting of LT-HSCs by CRISPR/Cas9. One of them is a transitory inhibition of intracellular pathways (e.g., p53 pathway), keeping the cells in the G0-G1 cell cycle stage. This transitory induction of cell cycling enables efficient gene-editing. At the same time, it is a matter of scientific discussion if short-term HSCs, or even more committed progenitors, may re-differentiate into LT-HSCs while receiving specific stimuli from, e.g., bone marrow microenvironment or feed-back humoral responses. Also, gene-edited hematopoietic cells acquire better fitness after correction of mutations and will have survival and proliferation advantage compared to uncorrected cells. *In vivo* delivery of the CRISPR/Cas9 complexes using an AAV serotype with tropism to HSCs or nanoparticles (ideally to LT-HSCs) is a matter of current scientific investigations. However, for the optimal *in vivo* delivery, the cell type- or even cell differentiation stage- specificity of AAV serotype and how to overcome immunological hurdles should be clarified. It remains an important issue to determine if CN patients have an existing, acquired immunity to Cas9 since they often suffer from severe bacterial infections until the optimal G-CSF dose is adjusted. Thus, one might assume that CN patients could have higher preexisting immunity to Cas9 than that described for healthy individuals.

It is also important to estimate the minimal effective therapeutic threshold of the gene-editing required to correct neutropenia phenotype and prevent the leukemogenic transformation in *HAX1-CN* patients. This might be achieved, although not optimally, in animal models using xenotransplantation of gene-edited cells invarious ratios to control edited cells. Alternatively, evaluation of the *in vivo* granulocytic differentiation of xenotransplanted primary HSPCs of *HAX1*-CN patients in various ratios to healthy donor-derived HSPCs should be performed in NSG mice. This might help estimate the optimal amount of gene-editing sufficient to support bone marrow and peripheral blood granulocytes' physiological levels.

Patient's age is also essential in gene therapy, given that *HAX1-CN* is a pre-leukemia disorder, and acquisition of pre- or even malignant HSCs clones with somatic leukemia-associated gene mutations might occur already at the early age of the patient's life. To avoid the possible leukemogenic transformation of HSCs after gene-editing, we need to evaluate patients' bone marrow using, e.g., ultra-deep-sequencing of a broad panel of leukemia-associated gene mutations. It will reduce the possibility of gene manipulation on already transformed HSCs, which may increase the chance of leukemogenic transformation. On the other side, the probability of developing leukemia will be dropped markedly after gene-edited correction of CN-causing mutations since no G-CSF therapy will be required anymore. It should be investigated whether HSCs with acquired mutations in CSF3R and/or *RUNX1* genes will still have clonogenic potential, even if they will not be permanently exposed to a high-dose of G-CSF anymore.

The inventors' platform may also be used to model *HAX1*-associated CN *in vitro* and *in vivo.* The ultimate mechanism of defective granulopoiesis downstream of the absent HAX1 expression is mostly unknown. There is no mouse model of *HAX1* deficiency (*Hax1*^{*-*/*-*} mice have normal neutrophil counts), and only sparse amounts of primary *HAX1-CN* patients' derived HSPCs are available for downstream investigations. The demonstrated here that the introduction of *HAX1* truncations in healthy donor-derived HSPCs led to diminished granulocytic differentiation *in vitro.* Thus, CRISPR/Cas9-based modeling of *HAX1*-CN using healthy donor-derived HSPCs might be used for downstream analysis to better understand granulopoiesis and leukemogenesis mechanisms.

6. Conclusion

## Claims

1. A 'single guide RNA' (sgRNA) molecule targeting the gene encoding HCLS1 Associated Protein X-1 (*HAX1* gene) in a living being for use in the prophylaxis and/or treatment and/or examination of a disease, **characterized in that** the sgRNA molecule comprises a nucleotide sequence which is selected from the group consisting of SEQ ID NOS: 1-553.

2. A 'repair template nucleic acid molecule' for correcting a mutation in the *HAX1* gene in a living being for use in the prophylaxis and/or treatment and/or examination of a disease, **characterized in that** the repair template nucleic acid molecule comprises the nucleotide sequence of SEQ ID NO: 565.

3. The sgRNA molecule of claim 1 or repair template nucleic acid molecule of claim 2, **characterized in that** said disease is congenital neutropenia, preferably severe congenital neutropenia (CN/SCN), and/or cyclic neutropenia (CyN).

4. The sgRNA molecule of claim 1 or repair template nucleic acid molecule of claim 2, **characterized in that** said disease is myelodysplastic syndrome (MDS) and/or myeloid leukemia (AML).

5. A vector comprising the sgRNA molecule of claims 1, 3 or 4 and/or the repair template nucleic acid molecule of claims 2-4.

6. The vector of claim 5, **characterized in that** said vector is a recombinant adeno-associated vector (rAAV), preferably with AAV6 serotype (rAAV6).

7. A composition comprising the sgRNA molecule of claims 1, 3 or 4 and/or the repair template nucleic acid molecule of claims 2-4 and/or the vector of claim 5 or 6.

8. The composition of claim 7, further comprising a CRISPR associated protein 9 (Cas9) and/or a vector encoding Cas9, preferably *Streptococcus pyogenes* Cas9 (S.p. Cas9), preferably S.p. Cas9 V3, and highly preferably S.p. HiFi Cas9 V3.

9. The composition of claim 7 or 8, which is a pharmaceutical composition comprising a pharmaceutically acceptable carrier.

10. A method *in vitro* for targeting the *HAX1* gene in biological material including genetic material encoding said *HAX1* gene, comprising the step of introducing the sgRNA molecule of claims 1, 3 or 4 and/or the repair template nucleic acid molecule of claims 2-4 and/or the vector of claim 5 or 6, and or said composition of any of claims 7-9 into said biological material, preferably said editing is made *via* CRISPR/Cas9 technology.

11. The method of claim 10, **characterized in that** said biological material comprises hematopoietic stem and progenitor cells (HSPCs).

12. A method for the prophylaxis and/or treatment and/or examination of a disease in a living being, comprising a step of targeting the *HAX1* gene in said living being by introducing the sgRNA molecule of claims 1, 3 or 4 and/or the repair template nucleic acid molecule of claims 2-4 and/or the vector of claim 5 or 6, and or said composition of any of claims 7-9 into said biological material, preferably said editing is made *via* CRISPR/Cas9 technology.

13. The method of claim 12, **characterized in that** said disease is selected from the group consisting of: congenital neutropenia, preferably severe congenital neutropenia (CN/SCN), cyclic neutropenia (CyN), myelodysplastic syndrome (MDS), myeloid leukemia (AML).

14. A method for editing and/or correcting in a cell a mutant allele of the *HAX1* gene, preferably having a mutation associated with congenital neutropenia, severe congenital neutropenia (CN/SCN) or cyclic neutropenia (CyN), and which *HAX1* gene of said cell is mutated at nucleotide position c.131insA, and/or which *HAX1* gene product is mutated at amino acid position p.W44X, the method comprising:
- introducing to the cell a composition comprising:
- a CRISPR associated protein 9 (Cas9) or a sequence encoding Cas9;
- an sgRNA molecule comprising a nucleotide sequence which is selected from the group consisting of SEQ ID NOS: 1-553;
wherein a complex of the Cas9 and the sgRNA molecule affects a double strand break in the mutant allele of the *HAX1* gene.

15. The method of claim 14, **characterized in that** said composition further comprises a repair template nucleic acid molecule comprising the nucleotide sequence of SEQ ID NO: 565, wherein said repair template nucleic acid molecule affects the repair of the mutant allele of the *HAX1* gene.
